(19) Europäisches Patentamt

European Patent Office

Office européen des brevets

(11) **EP 1 529 039 B1**

(12) **EUROPÄISCHE PATENTSCHRIFT**

(45) Veröffentlichungstag und Bekanntmachung des Hinweises auf die Patenterteilung:
**30.11.2005 Patentblatt 2005/48**

(21) Anmeldenummer: **03784039.4**

(22) Anmeldetag: **22.07.2003**

(51) Int Cl.$^7$: **C07D 307/60**

(86) Internationale Anmeldenummer:
**PCT/EP2003/007982**

(87) Internationale Veröffentlichungsnummer:
**WO 2004/014833 (19.02.2004 Gazette 2004/08)**

(54) **VERFAHREN ZUR HERSTELLUNG VON MALEINSAEUREANHYDRID**

METHOD FOR PRODUCING MALEIC ANHYDRIDE

PROCEDE DE PRODUCTION D'ANHYDRIDE D'ACIDE MALEIQUE

(84) Benannte Vertragsstaaten:
**AT BE BG CH CY CZ DE DK EE ES FI FR GB GR HU IE IT LI LU MC NL PT RO SE SI SK TR**

(30) Priorität: **02.08.2002 DE 10235355**

(43) Veröffentlichungstag der Anmeldung:
**11.05.2005 Patentblatt 2005/19**

(73) Patentinhaber: **BASF Aktiengesellschaft**
**67056 Ludwigshafen (DE)**

(72) Erfinder:
• **WEIGUNY, Jens**
**200336 Shanghai (CN)**
• **RUPPEL, Wilhelm**
**68163 Mannheim (DE)**
• **DUDA, Mark**
**67071 Ludwigshafen (DE)**

(56) Entgegenhaltungen:
**WO-A-01/68626        WO-A-02/12158**
**DE-A- 2 539 106**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft ein Verfahren zur Herstellung von Maleinsäureanhydrid durch heterogenkatalytische Gasphasenoxidation von Kohlenwasserstoffen mit mindestens vier Kohlenstoffatomen mit Sauerstoff enthaltenden Gasen in Gegenwart einer flüchtigen Phosphorverbindung an einem Vanadium, Phosphor und Sauerstoff enthaltenden Katalysator in einer Rohrbündelreaktor-Einheit mit mindestens einer durch ein Wärmeträgermedium gekühlten Reaktionszone bei einer Temperatur im Bereich von 350 bis 500°C.

Maleinsäureanhydrid ist ein wichtiges Zwischenprodukt bei der Synthese von γ-Butyrolacton, Tetrahydrofuran und 1,4-Butandiol, welche ihrerseits als Lösungsmittel eingesetzt werden oder beispielsweise zu Polymeren, wie Polytetrahydrofuran oder Polyvinylpyrrolidon weiterverarbeitret werden.

Die Herstellung von Maleinsäureanhydrid durch he terogenkatalytische Gasphasenoxidation von Kohlenwasserstoffen mit mindestens vier Kohlenstoffatomen mit Sauerstoff an einem Vanadium-, Phosphor- und Sauerstoff-enthaltendem Katalysator ist allgemein bekannt und beispielsweise in Ullmann' s Encyclopedia of Industrial Chemistry, 6th edition, 1999 Electronic Release, Chapter "MALEIC AND FUMARIC ACID - Maleic Anhydride" beschrieben. Im Allgemeinen werden Benzol oder $C_4$-Kohlenwasserstoffe, wie 1,3-Butadien, n-Butene oder n-Butan eingesetzt. Die Reaktion ist stark exotherm und bedarf einer ausreichenden Abfuhr der Reaktionswärme. Im Allgemeinen führt man die Umsetzung in einem Rohrbündelreaktor mit Salzkreislauf durch.

Eine wesentliche Zielsetzung bei der heterogenkatalytischen Gasphasenoxidationen von Kohlenwasserstoffen zu Maleinsäureanhydrid ist grundsätzlich die Erzielung einer möglichst hohe Raum-Zeit-Ausbeute an Maleinsäureanhydrid über die gesamte Katalysatorlebensdauer. Die Erreichung einer hohe Raum/Zeit-Ausbeute an Maleinsäureanhydrid ist dabei von verschiedenen Faktoren abhängig, wie beispielsweise von der Art des Katalysators, von dessen Aktivitätsverteilung im Katalysatorbett, von der Zugabe von Phosphorkomponenten zum Eduktgsinisch, von der Zusammensetzung des Eduktgemisches, von der Kohlenwasserstoffbelastung des Katalysators oder von der Reaktionstemperatur.

Da die Umsetzung der Kohlenwasserstoffe zu Maleinsäureanhydrid stark exotherm verläuft, bildet sich im Katalysatorbett im Allgemeinen ein Bereich erhöhter Temperatur ("Hot-Spot"-Bereich) aus, in dem durch eine Vielzahl möglicher Parallel- und Folgereaktionen die Selektivität zum und somit die Ausbeute an Wertprodukt vermindert ist. Um dem genannten Effekt entgegenzuwirken wurde in EP-A-0 099 431 vorgeschlagen, eine strukturierte, das heißt eine bezüglich der Aktivität angepaßte Katalysatorschüttung einzusetzen. Die niedrigste Katalysatoraktivität befindet sich am Reaktoreingang, die höchste am Reaktorausgang. Da zwischen kann sie kontinuierlich oder in Stufen variieren. Zur gezielten Einstellung der Katalystoraktivitäten lehrt die genannte Schrift im Wesentlichen die Verdünnung der aktiven Katalysatorpartikel mit Inertmaterial, den Einsatz verschieden aktiver Katalysatoren und gegebenenfalls Mischungen davon.

WO 93/01155 offenbart ein Verfahren zur Herstellung von Maleinsäureanhydrid aus n-Butan an einem Vanadium-, Phosphor- und Sauerstoff-enthaltenden Katalysator in Gegenwart einer flüchtigen Phosphorverbindung, bei dem die Katalysatoraktivität mit der Temperatur und der n-Butan-Konzentration in Fließrichtung des Gases so variiert, daß die Reaktionsgeschwindigkeit durch hohe Aktivität in einem Bereich von niedriger Temperatur und geringer n-Butan-Konzentration innerhalb des Bettes gefördert wird und durch eine niedrige Aktivität in einem kritischen Bereich innerhalb des Bettes, wo die Kombination aus Temperatur und n-Butan-Konzentration zu einem übermäßigen Ansteigen von Umsatz und Reaktionstemperatur führen würde, beschränkt wird.

Die oben genannte Strukturierung der Katalysatorschüttung führt zu einer gleichmäßigeren Temperaturverteilung und somit in der Regel auch zu einer Erhöhung der Raum/Zeit-Ausbeute an Maleinsäureanhydrid. Nachteilig ist jedoch die aufwändige Befüllung des Rohrbündelreaktors, da für jedes der bis zu mehreren zehntausend Reaktionsrohre die entsprechende Strukturierung beim Befüllen einzustellen ist.

Wellauer et al., Chem. Eng. Sci. Vol. 41, No. 4 (1986), Seite 765 bis 772, beschreiben ein Simulationsmodell zur Oxidation von n-Butan zu Maleinsäureanhydrid auf Basis experimenteller Daten eines bekannten Katalysators. Neben der bereits zuvor beschriebenen Strukturierung der Katalysatorschüttung lehren Wellauer et al. zur Erhöhung der Raum-Zeit-Ausbeute die Einstellung zweier unterschiedlicher Salzbadtemperaturen im Rohrbündelreaktor, wobei in der ersten, eduktseitigen Reaktionszone eine niedrigere Temperatur einzustellen ist als in der zweiten, produktseitigen Reaktionszone.

WO 01/68626 lehrt ein Verfahren zur Herstellung von Maleinsäureanhydrid aus n-Butan an einem Vanadium-, Phosphor- und Sauerstoff-enthaltenden Katalysator unter Einsatz eines Mehrzonen-Rohrbündelreaktors, bei dem die Temperaturdifferenz zwischen der heißesten und kältesten Reaktionszone mindestens 2°C beträgt.

Unabhängig vom möglichen Einsatz einer strukturierten Katalysatorschüttung oder eines Mehrzonenreaktors wird die Oxi dation von Kohlenwasserstoffen zu Maleinsäureanhydrid üblicherweise unter einem vorgewählten Druck und einer vorgewählten Gaszusammensetzung unter Einstellung der für eine maximale Ausbeute an Maleinsäureanhydrid erforderlichen Salzbadtemperatur betrieben. Dabei wird die Salzbadtemperatur in der Regel kontinuierlich oder sukzessive erhöht bis das Maximum der Maleinsäureanhydrid-Ausbeute erreicht ist. Beim Betrieb eines Mehrzonenreaktors werden im Allgemeinen die einzelnen Reaktionszonen nacheinander, beginnend bei der ersten, eduktseitigen Reaktionszone, über die Salzbadtemperatur auf maximale Maleinsäureanhydrid-Ausbeute eingestellt.

Erfindungsgemäß wurde erkannt, dass die oben beschriebene Einstellung der Salzbadtemperatur zur Erreichung des Maximums der Maleinsäureanhydrid-Ausbeute sicherheitstechnisch als sehr kritisch zu betrachten ist, da die daraus resultierende Salzbadtemperatur in einem Bereich liegen kann, in dem die Gefahr plötzlich auftretender, unkontrollierter Temperaturspitzen im Katalysatorbett besteht. Die genannten Temperaturspitzen können zu einer irreversiblen Schädigung des Katalysators führen. So besteht hierdurch die Gefahr schädlicher Veränderungen an der katalytisch aktiven Katalysatoroberfläche bis hin zur Sinterung und zum Verbacken des Katalysators. Dies wirkt sich auf die gesamte Katalysator-Performance, insbesondere auf die Aktivität, die Selektivität und die Katalysatorstandzeit, negativ aus. So kann beispielsweise eine Salzbadtemperatur, bei der anfänglich das Maximum der Maleinsäureanhydrid-Ausbeute erreicht wird infolge der zuvor geschilderten möglichen Schädigung des Katalysators zu einem relativ schnellen Abfall der Katalysatoraktivität und -selektivität führen und somit eine sehr kurze Katalysatorstandzeit nach sich ziehen.

Des Weiteren wurde erfindungsgemäß erkannt, dass durch die Gefahr plötzlich auftretender, unkontrollierter Temperaturspitzen im Katalysatorbett im Extremfall sogar das "Durchgehen" einzelner oder mehrerer Reaktionsrohre bis hin zum gesamten Rohrbündelreaktor, welcher gegebenenfalls mehrere zehntausend Reaktionsrohre enthalten kann, möglich ist, da im Bereich der genanzzten Temperaturspitzen die Reaktionsgeschwindigkeit stark ansteigt und somit an dieser Stelle noch mehr Wärme produziert wird. Dieser heiße Bereich kann sich in einem Rohrbündelreaktor über das Kühlmedium (Salzbad) auf Nachbarrohre bis hin zum gesamten Reaktorquerschnitt ausdehnen. Durch die heißen Temperaturen, welche bis zu 1000°C betragen können, kann im schlimmsten Fall sogar der gesamte Rohrbündelreaktor irreversibel geschädigt werden.

Die Aufgabe der vorliegenden Erfindung bestand darin, ein Verfahren zur Herstellung vom Maleinsäureanhydrid durch heterogenkatalytische Gasphasenoxidation eines Kohlenwasserstoffs mit mindestens vier Kohlenstoffatomen mit Sauerstoff zu entwickeln, welches in Bezug auf das Durchgehen der Reaktion sicherheitstechnisch unproblematisch ist und welches auch bei hoher Kohlenwasserstoff-Belastung des Katalysators über einen langen Zeitraum von mehreren Monaten bis einigen Jahren hinweg einen hohen Umsatz, eine hohe Selektivität sowie eine hohe Ausbeute an Wertprodukt und daher eine hohe Raum-Zeit-Ausbeute ermöglicht und eine frühzeitige Schädigung des Katalysators vermeidet oder zumindest stark mindert.

Demgemäß wurde ein Verfahren zur Herstellung von Maleinsäureanhydrid durch heterogenkatalytische Gasphasenoxidation von Kohlenwasserstoffen mit mindestens vier Kohlenstoffatomen mit Sauerstoff enthaltenden Gasen in Gegenwart einer flüchtigen Phosphorverbindung an einem Vanadium, Phosphor und Sauerstoff enthaltenden Katalysator in einer Rohrbündelreaktor-Einheit mit mindestens einer durch ein Wärmeträgermedium gekühlten Reaktionszone bei einer Temperatur im Bereich von 350 bis 500°C gefunden, das dadurch gekennzeichnet ist, dass man in der, in Bezug auf die Edukt-Zufuhr, ersten Reaktionszone die Zulauftemperatur und/oder die Zulaufmenge des Wärmeträgermediums derart einstellt, dass die mittlere Temperatur des Wärmeträgermediums in der ersten Reaktionszone $T_{SB}$ (1. Zone), welche sich durch Mittelwertbildung aus der Zulauftemperatur und der Ablauftamperatur des Wärmeträgermediums ergibt, die Formeln (I) und (II)

$$T_{SB}(1. \text{ Zone}) \leq T_D(1. \text{ Zone}) - T_{Safety} (1. \text{ Zone}) \qquad (I)$$

$$T_{SB,Amax}(1. \text{ Zone}) - T_A (1. \text{ Zone}) \leq T_{SB} (1. \text{ Zone}) \leq$$

$$T_{SB,Amax}(1. \text{ Zone}) + T_B(1. \text{ Zone}) \qquad (II)$$

erfüllt, wobei

$T_D$ (1. Zone) für die Durchgehtemperatur der ersten Reaktionszone steht, wobei diese der mittleren Temperatur des Wärmeträgermediums $T_{SB}$ (1. Zone) entspricht, bei der eine Erhöhung von einer um 1°C tieferen mittleren Temperatur des Wärmeträgermediums $T_{SB}$(1. Zone) - 1°C µm 1°C auf $T_{SB}$(1. Zone) eine Erhöhung der Heißpunkt-Temperatur in der ersten Reaktionszone $T_{HS}$(1- Zone) um 5°C bewirkt;

$T_{Safety}$(1. Zone) für die Sicherheitstemperatur der ersten Reaktionszone steht und 1°C beträgt;

$T_{SB,Amax}$(1. Zone) für die mittlere Temperatur des Wärmeträgermediums in der ersten Reaktionszone steht , bei der im Bereich von $T_{SB}$ (1. Zone) $\leq T_D$(1. Zone) das Maximum der Ausbeute an Maleinsäureanhydrid erreicht wird;

$T_A$ (1. Zone) 20°C beträgt; und

$T_B$ (1. Zone) 10°C beträgt.

Nach Formel (I) ist das Verfahren bei einer mittleren Temperatur des Wärmeträgermediums $T_{SB}$ (1. Zone) zu betreiben, welche mindestens um die sogenannte Sicherheitstemperatur $T_{Safety}$ (1. Zone) von der zuvor ermittelten Durchgehtemperatur $T_D$ (1. Zone) vermindert ist.

Die Durchgehtemperatur $T_D$ (1. Zone) entspricht dabei der mittleren Temperatur des Wärmaträgermediums $T_{SB}$ (1. Zone), bei der eine Erhöhung von einer um 1°C tieferen mittleren Temperatur des Wärmeträgermediums $T_{SB}$ (1. Zone) - 1°C um 1°C auf $T_{SB}$ (1. Zone) eine Erhöhung der Heißpunkt-Temperatur in der ersten Reaktionszone $T_{HS}$ (1. Zone) um 5°C bewirkt. Erfindungsgemäß wurde erkannt, dass bei einer Erhöhung der Heißpunkt-Temperatur in der ersten Reaktionszone $T_{HS}$ (1. Zone) um mehr als 5°C die Gefahr des "Durchgehens" einzelner oder mehrere Reaktionsrohre stark ansteigt.

Unter der Heißpunkt-Temperatur ist das innerhalb der befrachteten Reaktionszone befindliche Maximum der in der Katalysatorschüttung während der chemischen Reaktion gemessenen Temperatur zu verstehen.

Die Sicherheitstemperatur $T_{Safety}$(1. Zone) berücksichtigt dabei insbesondere die in einer technischen Rohrbündelreaktor-Einheit vorliegenden Inhomogenitäten, vor allem in Bezug auf den Katalysator und dessen Schüttung, auf die konkrete Belastung der einzelnen Rohre und die konkrete Wärmeabfuhr an den einzelnen Rohren durch das Wärmeträgermedium. Beim erfindungsgemäßen Verfahren beträgt die Sicherheitstemperatur $T_{Safety}$ (1. Zone) 1°C, bevorzugt 2°C, besonders bevorzugt 3°C und ganz besonders bevorzugt 4°C.

Formel (I) stellt somit sicher, dass selbst bei den zuvor genannten Inhomogenitäten in den technischen Rohrbündelreaktor-Einheiten der sicherheitsbedenkliche Bereich oberhalb der Durchgehtemperatur $T_D$ (1. Zone) nicht erreicht wird.

Um neben einer sicheren Reaktions führung auch eine hohe Ausbeute an Maleinsäureanhydrid zu erzielen, ist das Verfahren ferner nach Formel (II) bei einer mittleren Temperatur des Wärmeträgermediums $T_{SB}$(1. Zone) zu betreiben, welche in einem Bereich von $T_{SB,Amax}$ (1. Zone) - $T_A$ (1. Zone) bis $T_{SB,Amax}$ (1. Zone) + $T_B$ (1. Zone) liegt, wobei für $T_{SB,Amax}$ (1. Zone) das Maximum der Ausbeute an Maleinsäureanhydrid im Bereich $\leq T_D$ (1. Zone) heranzuziehen ist. $T_{SB,Amax}$ (1. Zone) entspricht dabei der mittleren Temperatur des Wärmeträgermediums in der ersten Reaktionszone, bei der im Bereich von $T_{SB}$ (1. Zone) $\leq T_D$ (1. Zone) das Maximum der Ausbeute an Maleinsäureanhydrid erreicht wird.

Beim erfindungsgemäßen Verfahren beträgt der Parameter $T_A$(1. Zone) 20°C, bevorzugt 10°C und besonders bevorzugt 5°C und der Parameter $T_B$ (1. Zone) 10°C, bevorzugt 7°C und besonders bevorzugt 5°C.

Formel (II) stellt somit sicher, dass zusätzlich zur sicheren Reaktionsführung auch eine hohe Ausbeute an Maleinsäureanhydrid erreicht wird.

Die beim erfindungsgemäßen Verfahren einzustellende mittlere Temperatur des Wärmeträgermediums $T_{SB}$ (1. Zone) muß sowohl Formel (I) als auch Formel (II) erfüllen, was der Bildung einer Schnittmenge für den einzustellenden Bereich für $T_{SB}$(1. Zone) gleichzusetzen ist.

Wie aus der Definition von $T_{SB,Amax}$ (1. Zone), der mittleren Temperatur des Wärmeträgermediums in der ersten Reaktionszone, bei der das Maximum der Ausbeute an Maleinsäureanhydrid erreicht wird, hervorgeht, ist hierfür nur der Bereich $\leq T_D$(1. Zone) heranzuziehen. Steigt beispielsweise die Ausbeute an Maleinsäureanhydrid im untersuchten Bereich von $\leq T_D$(1. Zone) stetig an ohne ein Maximum zu durchlaufen, so ist in Formel (II) als $T_{SB,Amax}$(1. Zone) die Durchgehtemperatur $T_D$(1. Zone) einzusetzen.

Wurde beispielsweise die mittlere Temperatur des Wärmeträgermediums in der ersten Reaktionszone $T_{SB,Amax}$ (1. Zone), bei der das Maximum der Ausbeute an Maleinsäureanhydrid im Bereich $\leq T_D$ (1. Zone) erreicht wird, bestimmt, jedoch nicht die Durchgehtemperatur $T_D$ (1. Zone), da diese gegebenenfalls bei einer deutlich höheren Temperatur liegen kann als $T_{SB,Amax}$ (1. Zone), so ist für die Durchgehtemperatur $T_D$ (1. Zone) in Formel (I) sicherheitshalber die maximale Temperatur $T_{SB}$ (1. Zone) der vorliegenden Daten einzusetzen. Diese liegt dann mit Sicherheit unterhalb der wahren Durchgehtenperatur $T_D$ (1. Zone).

Die Bestimmung der mittleren Temperatur des Wärmeträgermediums $T_{SB}$ (1. Zone) erfolgt durch Mittelwertbildung aus der Zulauftemperatur und der Ablauftemperatur des Wärmeträgermediums.

Die gezielte Einstellung der mittleren Temperatur des Wärmeträgermediums $T_{SB}$ (1. Zone) erfolgt beim erfindungsgemäßen Verfahren durch eine gezielte Einstellung der Zulauftemperatur und/oder der Zulaufmenge des Wärmeträgermediums .

Bei der Ermittlung der Durchgehtemperatur $T_D$ (1. Zone) und der mittleren Temperatur des Wärmeträgermediums $T_{SB, Amax}$ (1. Zone), bei der im Bereich von $T_{SB}$ (1. Zone) $\leq T_D$ (1. Zone) das Maximum der Ausbeute an Maleinsäureanhydrid erreicht wird, sind die beim durchzuführenden Verfahren vorliegenden Bedingungen zu berücksichtigen. Im Allgemeinen erfolgt die Ermittlung experimentell im einer Versuchsanlage unter Einsatz des entsprechenden Katalysators, wobei die für das Reaktionsverhalten relevanten Parameter wie beispielsweise der innere Durchmesser des Reaktionsrohres (beziehungsweise der Reaktiorisrohre), der Druck, die Kohlenwasserstoff-Konzentration, die GHSV, die Konzentration an der flüchtigen Phosphorverbindung und die Konzentrationen eventueller weiterer Zusätze wie etwa Wasserdampf einzustellen sind. Sofern die Abmessungen des in der Versuchsanlage verwendeten Reaktorrohres im Bereich der technisch verwendeten Reaktorrohre liegen, ist ein von einem Wärmeträgermedium umgebenes Reaktorrohr für die erforderliche Ermittlung von $T_D$(1. Zone) in der Regel sehr gut geeignet.

Bei der experimentellen Ermittlung von $T_D$ (1. Zone) und $T_{SB,Amax}$ (1. Zone) fährt man im Allgemeinen den Versuchsreaktor unter analogon Bedingungen an wie den später einzusetzenden Reaktor. In der Regel stellt man am Ende der Einfahrperiode eine mittlere Temperatur des Wärmeträgermediums $T_{SB}$(1. Zone) ein, welche deutlich unter der erwarteten Durchgehtemperatur $T_D$(1. Zone) liegt und dennoch eine Ausbeute an Maleinsäureanhydrid A im technisch relevanten Bereich liefert. Da die Heißpunkt-Temperatur $T_{HS}$(1. Zone) eine wesentliche Größe bei der Ermittlung der Durchgehtemperatur $T_D$(1. Zone) darstellt, ist besonderes Augenmerk auf einen stabilen Betriebspunkt zu legen. Im Rahmen des vorliegenden. Verfahrens wird von einem stabilen Betriebspunkt, ausgegangen, wenn unter konstanten Reaktionsbedingungen innerhalb von 24 Stunden die Drift der Heißpunkt-Temperatur $T_{HS}$ (1. Zone) $\leq$ 0,5°C beträgt. Nach der Ermittlung der zur eingestellten Heißpunkt-Temperatur $T_{SB}$ (1. Zone) ausgebildeten Heißpunkt-Temperatur $T_{HS}$ (1. Zone) sowie der Ausbeute an Maleinsäureanhydrid wird die mittlere Temperatur des Wärmeträgermediums $T_{SB}$ (1. Zone) sukzessive erhöht und jeweils nach Einstellung eines stabilen Betriebspunktes die entsprechende Heißpunkt-Temperatur $T_{HS}$ (1. Zone) und die Ausbeute an Maleins äureanhydrid ermittelt. Falls eine Erhöhung der mittleren Temperatur des Wärmeträgermediums $T_{SB}$ (1. Zone) um 1°C eine Erhöhung der Heißpunkt-Temperatur $T_{HS}$ (1. Zone) von mehr als 5°C bewirkt, kann die Versuchsreihe in der Regel abgebrochen werden.

Die Gasphasenoxidation zu Maleinsäureanhydrid erfolgt beim erfindungsgemäßen Verfahren in einer Rohrbündelreaktor-Einheit mit mindestens einer durch ein Wärmeträgermedium gekühlten Reaktionszone. Unter dem Begriff Rohrbündelreaktor-Einheit ist eine Einheit aus mindestens einem Rohrbündelreaktor zu verstehen. Ein Rohrbündelreaktor besteht wiederum aus mindestens einem Reaktorrohr, welches zur Beheizung und/oder Kühlung von einem Wärmeträgermedium umgeben ist. Im Allgemeinen enthalten die technisch eingesetzten Rohrbündelreaktoren wenige hundert bis mehrere zehntausend parallel-geschaltete Reaktorrohre. Sind mehrerer einzelne Rohrbündelreaktoren (im Sinne von Rohrbündelreaktor-Apparaten) parallel-geschaltet, so sind diese als Äquivalent eines Rohrbündelreaktors zu verstehen und im Folgenden im Begriff Rohrbündelreaktor enthalten.

Die Rohrbündelreaktor-Einheit kann eine oder mehrere Vorheizzonen enthalten, welche das eintretende Gasgemisch aufheizen. Eine in einem Rohrbündelreaktor integrierte Vorheizzone kann beispiel s-weise durch mit inertmaterial gefüllte Reaktorrohre, welche ebenfalls von Wärmeträgermedium umgeben sind, realisiert werden. Als Inertmaterial sind prinzipiell alle Formkörper geeignet, welche chemisch inert sind, d.h. keine heterogenkatalytische Reaktion induzieren oder katalysieren, und welche einen maximalen Druckverlust unterhalb des jeweiligen, maximal tolerierbaren, anlagenspezifischen Wert aufweisen. Geeignet sind beispielsweise oxidische Materialen, wie etwa Aluminiumoxid, Siliciumcarbid oder metallische Materialien, wie etwa Edelstahl. Als Formkörper seien beispielsweise Kugeln, Tabletten, Hohlzylinder, Ringe, Trilobes, Tristars, Wagenräder, Extrudate oder regellos gebrochene Formkörper genannt.

Besteht die Rohrbündelreaktor-Einheit aus mehreren Rohrbündelreaktoren, beispielsweise zwei, drei, vier oder mehr, so können diese beispielsweise parallelgeschaltet oder hintereinandergeschaltet vorliegen. Bei einer Hintereinanderschaltung von Rohrbündelreaktoren wird der Ausgangsstrom des einen Rohrbündelreaktors direkt in den Eingang des folgenden Rohrbündelreaktors geleitet . Es ist aber auch möglich, zwischen den beiden Rohrbündelreaktoren Masse und/oder Energie ab- und/oder zuzuführen. So kann beispielsweise ein Teil des Gasstroms oder eine Komponente davon entnommen oder ein weiterer Gasstrom zugeführt werden oder der vorhandene Gasstrom durch einen Wärmetauscher geleitet werden.

Üblicherweise sind in den vorgenannten Rohrbündelreaktoren die Reaktorrohre aus ferritischem Stahl gefertigt und weisen in typischer Weise eine Wanddicke von 1 bis 3 mm auf. Ihr Innendurchmesser beträgt in der Regel 20 bis 30 mm. Die Anzahl der Reaktorrohre je Rohrbündelreaktor liegt üblicherweise im Bereich zwischen 5000 und 35000, obgleich auch eine Anzahl über 35000 bei besonders großen Anlagen realisiert werden kann. Innerhalb des Reaktorkorpus sind die Reaktorrohre im Normalfall homogen verteilt angeordnet.

Unter dem Begriff Reaktionszone ist ein Bereich innerhalb eines Rohrbündelreaktors zu verstehen, welcher einen Katalysator enthält und bei dem die Temperatur infolge des umgebenden Wärmeträgermediums bei Abwesenheit einer chemischen Reaktion auf einen einheitlichen Wert gehalten werden würde. Im Allgemeinen wird die Reaktionszone durch die lokale Ausdehnung des Wärmeträgerkreislaufs abgegrenzt. So umfasst beispielsweise ein Rohrbündelreaktor mit nur einem Wärmeträgerkreislauf auch nur eine Reaktionszone, welche konventionsgemäß als erste Reaktionszone bezeichnet wird. Besteht eine Rohrbündelreaktor-Einheit beispielsweise aus einem Rohrbündelreaktor mit zwei getrennten, nachfolgenden Wärmeträgerkreisläufen, so umfasst dieser zwei Reaktionszonen, wobei die Numerierung der Reaktionszonen entsprechend der Durchlei tungsrichtung des Gases erfolgt.

Als Wärmeträgermedien eignen sich insbesondere fluide Temperiermedien. Besonders günstig ist die Verwendung von Salzschmelzen, wie Kaliumnitrat, Kaliumnitrit, Natriumnitrat und/oder Natriumnitrit oder von niedrig schmelzenden Metallen wie Natrium sowie Legierungen verschiedener Metalle.

Beim erfindungsgemäßen Verfahren setzt man bevorzugt eine Rohrbündelreaktor-Einheit mit mindestens zwei durch ein Wärmeträgermedium gekühlten Reaktionszonen ein. Durch die gezielte Einstellung der mittleren Temperatur des Wärmeträgermediums $T_{SB}$(1. Zone) in der ersten Reaktionszone gemäß der Formeln (I) und (II) wird in der, infolge der vorliegenden hohen Kohlenwasserstoff-Konzentration sicherheitstechnisch besonders sensiblen Zone

5

ein sicherer Betrieb ermöglicht. Durch die getrennten Reaktionszonen wird eine, dem Reaktionsverlauf angepaßte Reaktionsführung möglich, welche im Allgemeinen eine höhere Ausbeute an Maleinsäureanhydrid und eine flexiblere Regelung des Verfahrens erlaubt als der Einsatz eines Einzonenreaktors.

Beim bevorzugten Einsatz einer Rohrbündelreaktor-Einheit mit mindestens zwei durch ein Wärmeträgermedium gekühlten Reaktionszonen stellt man beim erfindungsgemäßen Verfahren besonders bevorzugt die Zulauftemperatur und/oder die Zulaufmenge des Wärmeträgermediums der, in Bezug auf die Edukt-Zufuhr, zweiten Reaktionszone derart ein, dass die mittlere Temperatur des Wärmeträgermediums in der zweiten Reaktionszone $T_{SB}$(2. Zone), welche sich durch Mittelwertbildung aus der Zulauftemperatur und der Ablauftemperatur des Wärmeträgermediums ergibt, die Formeln (III) und (IV)

$$T_{SB}(2\text{ - Zone}) \leq T_D(2.\text{ Zone}) - T_{Safety}(2.\text{ Zone}) \qquad (III)$$

$$T_{SB,Amax}(2.\text{ Zone}) - T_A(2\text{- Zone}) \leq T_{SB}(2.\text{ Zone}) \leq$$

$$T_{SB,Amax}(2.\text{ Zone}) + T_B(2.\text{ Zone}) \qquad (IV)$$

erfüllt, wobei

$T_D$(2. Zone) für die Durchgehtemperatur der zweiten Reaktionszone steht, wobei diese der mittleren Temperatur des Wärmeträgermediums $T_{SB}$(2. Zone) entspricht, bei der eine Erhöhung von einer um 1°C tieferen mittleren Temperatur des Wärmeträgermediums $T_{SB}$(2. Zone) - 1°C um 1°C auf $T_{SB}$(2. Zone) eine Erhöhung der Heißpunkt-Temperatur in der zweiten Reaktionszone $T_{HS}$ (2. Zone) um 5°C bewirkt;

$T_{Safety}$(2. Zone) für die Sicherheitstemperatur der zweiten Reaktionszone steht und 1°C beträgt;

$T_{SB,Amax}$ (2. Zone) für die mittlere Temperatur des Wärmeträgermediums in der zweiten Reaktionszone steht, bei der im Bereich von $T_{SB}$(2. Zone) ≤ $T_D$ (2. Zone) das Maximum der Ausbeute an Maleinsäureanhydrid erreicht wird;

$T_A$ (2. Zone) 10°C beträgt; und

$T_B$(2. Zone) 10°C beträgt.

Dadurch wird durch Formel (III) auch für die zweite Reaktionszone sichergestellt, dass bei auftretenden Inhomogenitäten in den technischen Rohrbündelreaktor-Einbeiten der sicherheitsbedenkliche Bereich oberhalb der Durchgehtemperatur $T_D$ (2. Zone) nicht erreicht wird. Ferner wird durch Formel (IV) sichergestellt, dass zusätzlich zur sicheren Reaktionsführung auch eine hohe Ausbeute an Maleinsäureanhydrid erreicht wird.

Auch für die zweite Reaktionszone ist das in Bezug auf die Formeln (I) und (II) zuvor beschriebene prinzipiell gültig. So ist beispielsweise die mittlere Temperatur des Wärmeträgermediums $T_{SB}$(2. Zone) derart einzustellen, dass sowohl Formel (I) als auch Formel (II) erfüllt werden.

Beim besonders bevorzugten erfindungsgemäßen Verfahren beträgt die Sicherheitstemperatur $T_{Safety}$ (2. Zone) 1°C, bevorzugt 2°C, besonders bevorzugt 3°C und ganz besonders bevorzugt 4°C. Der Parameter $T_A$ (2. Zone) beträgt 20°C, bevorzugt 10°C und besonders bevorzugt 5°C und der Parameter $T_B$(2. Zone) 10°C, bevorzugt 7°C und besonders bevorzugt 5°C.

Bei der Ermittlung der Durchgehtremperatur $T_D$ (2. Zone) und der mittleren Temperatur des Wärmeträgermediums $T_{SB,Amax}$(2, Zone), bei der im Bereich von $T_{SB}$(2. Zone) ≤ $T_D$ (2. Zone) das Maximum der Ausbeute an Maleinsäureanhydrid erreicht wird, ist zuvor die erste Reaktionszone entsprechend der zuvor ermittelten Fahrweise unter Erfüllung der Formeln (I) und (II) einzustellen. Die Ermittlung von $T_D$(2. Zone) und $T_{SB,Amax}$(2. Zone) erfolgt dann im Allgemeinen analog der bereits zuvor beschriebenen Ermittlung von $T_D$(1. Zone) und $T_{SB,Amax}$(1. Zone), worauf hiermit verwiesen sei.

Beim Einsatz einer Rohrbündelreaktor-Einheit mit mindestens zwei durch ein Wärmeträgermedium gektihlten Reaktionszonen hat es sich als besonders vorteilhaft herausgestellt, die Zulauftemperatur und/oder die Zulaufmenge des Wärmeträgermediums der zweiten Reaktionszone derart einzustellen, dass die Heißpunkt-Temperatur der zweiten Reaktionszone $T_{HS}$ (2. Zone) höher ist als die Heißpunkt-Temperatur der ersten Reaktionszone $T_{HS}$ (1. Zone). Hierdurch wird eine besonders hohe Ausbeute an Maleinsäureanhydrid erreicht.

Bevorzugt liegt die Heißpunkt-Temperatur der zweiten Reaktionszone $T_{HS}$(2. Zone) um mindestens 1°C, besonders bevorzugt um mindestens 2°C, ganz besonders bevorzugt um mindestens 4°C und insbesondere um mindestens 6°C höher als die Heißpunkt-Temperatur der ersten Reaktionszone $T_{HS}$ (1. Zone).

Des Weiteren ist es beim erfindunsgemäßen Verfahren vorteilhaft in mindestens einer der Reaktionszonen eine, hinsichtlich der Aktivität, strukturierte Katalysatorschüttung einzusetzen. Diese weist üblicherweise in einem Bereich niedriger Temperatur und geringer Kohlenwasserstoff-Konzentration eine hohe Aktivität und in einem Bereich, in dem durch das Zusammenwirken von Temperatur und der vorliegenden Kohlenwasserstoff-Konzentration ein übermäßiges-Ansteigen der Reaktionsgeschwindigkeit und der Temperatur hervorrufen könnte, eine niedrige Aktivität auf. Im Allgemeinen ist hierbei die Aktivität im Bereich der Heißpunkt-Temperatur gegenüber der verbleibenden Katalysatorschüttung herabzusetzen.

Die Strukturierung der Katalysatorschüttung kann durch verschiedene Maßnahrnen, gegebenenfalls in deren Kombination, erreicht werden. So ist es beispielsweise möglich, den Katalysator mit Inertmaterial, zum Beispiel mit Formkörpern aus Steatit, Aluminiumoxid, Siliciumcarbid oder einem anderen inerten Material, zu verdünnen. Ferner ist auch eine Aktivitätsstrukturierumg durch den Einsatz unterschiedlich aktiver Katalysatoren möglich. Diese wiederum kann durch eine unterschiedliche Formgebung und/oder durch den Einsatz unterschiedlicher Aktivinassen erreicht werden.

Die beim erfindungsgemäßen Verfahren einsetzbaren Vanadium, Phosphor und Sauerstoff enthaltenden Katalysatoren umfassen als katalytisch aktive Masse eine sauerstoffhaltige Vanadium-Phosphor-Verbindung oder Gemische solcher Verbindungen. Geeignete Aktivmassen sind beispielsweise in den Patentschriften US 5,275,996, US 5,641,722, US 5,137,860, US 5,095,125 oder US 4,933,312 beschrieben.

Sie können des weiteren sogenannte Promotoren enthalten. Als geeignete Promotoren sind die Elemente der 1. bis 15. Gruppe des Periodensystems sowie deren Verbindungen genannt. Geeignete Promotoren sind beispielsweise in den Offenlegumgsschriften WO 97/12674 und WO 95/26817 sowie in den Patenten US 5, 137, 860, US 5, 296, 436, US 5, 158, 923 und US 4, 795, 818 beschrieben. Bevorzugt werden als Promotoren Verbindungen der Elemente Kobald, Molybdän, Eisen, Zink, Hafnium, Zirkon, Lithium, 'Titan, Chrom, Mangan, Nickel, Kupfer, Bor, Silicium, Antimon, Zinn, Niob und Wismut, besonders bevorzugt Molybdän, Eisen, Zink, Antimon, Wismut, Lithium. Die promotierten Katalysatoren können einen oder mehrere Promotoren enthalten. Der Gehalt an Promotoren beträgt in Summe im fertigen Katalysator im Allgemeinen nicht mehr als etwa 5 Gew.-%, jeweils als Oxid gerechnet.

Bei der Herstellung der Katalysatoren können ferner auch sogenannte Hilfsmittel, wie etwa Tablettierhilfsmittel oder Porenbildner eingesetzt werden.

Tablettierhilfsmittel werden im Allgemeinen zugesetzt, wenn die Formgebung der erf indungs gemäßen Katalysatoren über eine Tablettierung erfolgt. Tablettierhilfsmittel sind in der Regel katalytisch inert und verbessern die Tablettiereigenschaften des sogenannten Precursorpulvers, einer Zwischenstufe in der Katalysatorherstellung, beispielsweise durch Erhöhung der Gleit- und Rieselfähigkeit. Als geeignetes und bevorzugtes Tablettierhilfsmittel sei Graphit genannt. Die zugesetzten Tablettierhilfsmittel verbleiben in der Regel im aktivierten Katalysator. Typischerweise liegt der Gehalt an Tabletti erhilfsmittel im fertigen Katalysator bei etwa 2 bis 6 Gew.-%.

Porenbildner sind Stoffe, welche zur gezielten Einstellung der Porenstruktur im Makroporenbereich eingesetzt werden. Sie können prinzipiell unabhängig vorn Formgebungsverfahren eingesetzt werden. In der Regel handelt es sich um Kohlenstoff, Wasserstoff, Sauerstoff und/oder Stickstoff enthaltende Verbindungen, welche vor der Formgebung des Katalysator zugesetzt werden und bei der anschließenden Aktivierung des Katalysators unter Sublimation Zersetzung und/oder Verdampfung zum überwiegenden Teil wieder entfernt werden. Der fertige Katalysator kann dennoch Rückstände oder Zersetzungsprodukte des Porenbildners enthalten.

Die beim erfindungsgemäßen Verfahren einsetzbaren Katalysatoren können die Aktivmasse beispielsweise in reiner, unverdünnter Form als sogenannter "Vollkatalysator" oder verdünnt mit einem bevorzugt oxidischen Trägermaterial als sogenannter "Mischkatalysator" enthalten. Als geeignete Trägermaterialien für Mischkatalysatoren seien bei spielsweise Aluminiumoxid, Siliciumdioxid, Alumosilikate, Zirkondioxid, Titandioxid oder Gemische davon genannt. Bevorzugt ist die Herstellung von Voll- und Mischkatalysatoren, besonders bevorzugt von Vollkatalysatoren.

Der beim erfindungsgemäßen. Verfahren bevorzugt einzusetzende Katalysator weist Partikel mit einem gemittelten Durchmesser von mindestens 2 mm, bevorzugt mindestens 3 mm auf. Unter dem gemittelten Durchmesser eines Partikels ist der Mittelwert aus der kleinsten und der größten Abmessung zwischen zwei planparallelen Platten zu verstehen.

Unter Partikeln sind sowohl regellos geformte Partikel als auch geometrisch geformte Partikel, sogenannte Formkörper, zu verstehen. Bevorzugt weist der beim erfindungsgemäßen Verfahren einzusetzende Katalysatorprecursor Formkörper auf. Als geeignete Formkörper seien beispielsweise genannt Tabletten, Zylinder, Hohlzylinder, Kugeln, Stränge, Wagenräder oder Extrudate. Besondere Formen, wie beispielsweise "Trilobes" und "Tristars" (siehe EP-A-0 593 646) oder Formkörper mit mindestens einer Einkerbung an der Außenseite (siehe US 5,168,090) sind ebenfalls möglich.

Besonders bevorzugt weist der beim erfindungsgemäßen Verfahren einsetzbare Katalysator Formkörper mit seiner im Wesentlichen hohlzylinderförmigen Struktur auf. Unter einer im wesentlichen hohlzylinderförmigen Struktur ist eine Struktur zu verstehen, welche im wesentlichen einen Zylinder mit einer zwischen den beiden Deckelflächen hindurchgehenden Öffnung umfaßt. Der Zylinder ist charakterisiert durch zwei im Wesentlichen parallele Deckelflächen und einer Mantelfläche, wobei der Querschnitt des Zylinders, d.h. parallel zu den Deckelflächen, im Wesentlichen von kreisförmiger Struktur ist. Der Querschnitt der hindurchgehenden Öffnung, d.h. parallel zu den Deckelflächen des zylinders, ist im Wesentlichen ebenfalls von kreisförmiger Struktur. Bevorzugt befindet sich die hindurchgehende Öffnung mittig zu, den Deckelflächen, wobei andere räumliche Anordnungen damit nicht ausgeschlossen sind.

Der Begriff "im Wesentlichen" weißt darauf hin, dass Abweichungen von der Idealgeometrie, wie beispielsweise leichte Deformationen der kreisförmigen Struktur, nicht planparallel ausgerichtete Dekkelflächen, abgeplatzte Ecken und Kanten , Oberflächenrauhigkeit oder Einkerbungen in der Mantelfläche, den Deckelflächen oder der Innenfläche der hindurchgehenden Bohrung beim Katalysatorprecursor mit umfasst sind. Im Rahmen der Genauigkeit der Tablettierkunst sind kreisförmige Deckelflächen, ein kreisförmiger Querschni tt der hindurchgehenden Bohrung, parallel ausgerichtete Dekkelflächen und makroskopisch glatte Oberflächen bevorzugt.

Die im Wesentlichen hohlzylinderförmige Struktur kann beschrieben werden durch einen äußeren Durchmesser $d_1$, einer Höhe h als Abstand der beiden Deckel flächen und einem Durchmesser des inneren Lochs (hindurchgehende Öffnung) $d_2$. Der äußere Durchmesser $d_1$ des Katalysatorprecursors beträgt bevorzugt 3 bis 10 mm, besonders bevorzugt 4 bis 8 mm, ganz besonders bevorzugt 4,5 bis 6 mm. Die Höhe h beträgt bevorzugt 1 bis 10 mm, besonders bevorzugt 2 bis 6 mm, ganz besonders bevorzugt 2 bis 4 mm. Der Durchmesser der hindurchgehenden Öffnung $d_2$ beträgt bevorzugt 1 bis 8 mm, besonders bevorzugt 2 bis 6 mm, ganz besonders bevorzugt 2 bis 3,5 mm.

Die Katalysatorherstellung ist im Allgemeinen ein mehrstufiger Prozeß, bei dem man zunächst eine sogenannten Katalysatorprecursor herstellt und diesen anschließend durch Kalzinierung in die aktive Form überführt. Die beim erfindungsgemäßen Verfahren einsetzbaren Katalysatorprecursor können beispielsweise wie im den Patentschriften US 5,275, 996 und US 5,641,722 oder der Offenlegungsschrift WO 97/12674 beschrieben hergestellt werden. Die wesentlichen Schritte einer bevorzugten Herstellung des Katalysatorprecursors sind im Folgenden erläutert.

(a) Umsetzung einer fünfwertigen Vanadiumverbindung (z.B. $V_2O_5$) und gegebenenfalls einer Promotorkomponente (z.B. $MoO_3$) mit einem organischen, reduzierenden Lösungsmittel (z.B. Alkohol, wie etwa Isobutanol) in Gegenwart einer fünfwertigen Phosphorverbindung (z.B. Ortho- und/oder Pyrophosphorsäure, Phosphorsäure ester) und/oder einer dreiwertigen Phosphorverbindung (z.B. phosphorige Säure) unter Erwärmen. Gegebenenfalls kann diese Stufe in Gegenwart eines dispergierten, pulverförmigen Trägermaterials durchgeführt werden. Bevorzugt ist die Umsetzung ohne Zusatz von Trägermaterial.

(b) Isolierung des gebildeten vanadium-, Phosphor-, Sauerstoff und gegebenenfalls Promotor enthaltenden katalysatorprecursors ("VPO-Precursor"), z.B. durch Filtration oder Eindampfung.

(c) Trocknung des VPO-Precursors und bevorzugt beginnende Präformierung durch Temperung bei einer Temperatur von 250 bis 350°C. Dem getrockneten und bevorzugt getemperten VPO-Precursor-Pulver kann nun gegebenenfalls pulverförmiges Trägermaterial und/oder ein sogenannter Porenbildner, wie beispielsweise Stearinsäure, CeLlulose oder Paraffine untermischt werden. Bevorzugt ist die Weiterverarbeitung ohne Zusatz eines Trägermaterials sowie ohne Zusatz eines Porenbildners.

(d) Formgebung durch Überführung in die gewünschte Struktur, bevorzugt in die im Wesentlichen hohlzylinderförmige Struktur. Die Formgebung erfolgt bevorzugt durch Tablettierung, vorteilhafterweise unter vorheriger Untermischung- eines sogenannten Gleitmittels, wie etwa Graphit.

Als weniger bevorzugte Alternative zur Tablettierung sei beispielsweise die Extrusion genannt. Bei dieser Variante teigt man beispielsweise den in (b) erhaltenen VPO-Precursor an, um eine extrusionsfähige Masse zu erhalten. Diese kann dann in die gewünschte Struktur extrudiert und zum Erhalt des einsetzbaren Katalysatorprecursors getrocknet werden.

Die Kalzinierung des Katalysatorprecursors erfolgt im Allgemeinen in Gegenwart einer Atmosphäre, enthaltend Sauerstoff, Wasserstoffoxid (Wasserdampf) und/oder Inertgas in einem Temperaturbereich von 250 bis 600°C. Als geeignete Inertgase seien beispielsweise Stickstoff, Kohlendioxid und Edelgase genannt. Bevorzugt werden bei der Kalzinierung nach dem erfindungsgemäßen Verfahren mindestens zwei Kalzinierungszonen, beispielsweise zwei bis zehn Kalzinierungszonen, mit unterschiedlicher Gasatmosphäre und gegebenenfalls unterschiedlicher Temperatur durchlaufen . Durch geeignete, an das jeweilige Katalysatorsystem angepaßte Kombination von Temperaturen, Behandlungsdauern und Gasatmosphären kann die mechanische und katalytische Eigenschaft des Katalysators beeinflußt und somit gezielt eingestellt werden.

Bevorzugt ist eine Kalzinierung, bei der man den Katalysatorprecursor

(a) in mindestens einer Kalzinierungszone in einer oxidierenden Atmosphäre mit einem Sauerstoff-Gehalt von 2 bis 21 Vol.-% auf eine Temperatur von 200 bis 350°C aufheizt und unter diesen Bedingungen bis zur Einstellung der gewünschten mittleren Oxidationsstufe des Vanadiums beläßt; und

(b) in mindestens einer weiteren Kalzinierungszone in einer nicht-oxidierenden Atmosphäre mit einem Sauerstoff-Gehalt von ≤ 0,5 Vol.-% und einem Wasserstoffoxid-Gehalt von 20 bis 75 Vol.-% auf eine Temperatur von 300 bis 500°C aufheizt und ≥ 0,5 Stunden unter diesen Bedingungen beläßt.

Bei Schritt (a) wird der Katalysatorprecursor in einer oxidierend wirkenden Atmosphäre mit einem Gehalt an molekularem Sauerstoff von im Allgemeinen 2 bis 21 Vol.-% und bevorzugt von 5 bis 21 Vol.-% bei einer Temperatur von 200 bis 350°C und bevorzugt von 250 bis 350°C über einen Zeitraum, der wirksam ist, die gewünschte mittlere Oxidationsstufe des Vanadiums einzustellen, belassen. Im Allgemeinen setzt man bei Schritt (a) Mischungen aus Sauerstoff, Inertgasen (z.B. Stickstoff oder Argon), Wassersto ffoxid (Wasserdampf) und/oder Luft sowie Luft ein. Aus der Sicht des durch die Kalzinierungszone (n) geführten Katalysatorprecursors kann die Temperatur während des Kalzinierschrittes (a) konstant gehalten werden, im Mittel steigen oder fallen. Da dem Schritt (a) im Allgemeinen eine Aufheizphase vorangeschaltet ist, wird die Temperatur in der Regel zunächst ansteigen, um dann bei dem gewünschten Endwert einzupendeln. Im Allgemeinen ist daher der Kalzinierungszone von Schritt (a) mindestens eine weitere Kalzinierungszone zur Aufheizung des Katalysatorprecursors vorangeschaltet.

Der Zeitraum, über den die Temperung in Schritt (a) aufrecht erhalten wird, ist beim erfindungsgemäßen Verfahren bevorzugt derart zu wählen, dass sich eine mittlere Oxidationsstufe des Vanadiums auf einen Wert von +3,9 bis +4,4, bevorzugt von +4,0 bis +4,3, einstellt. Die Bestimmung der mittleren Oxidationsstufe des Vanadiums erfolgt über potentiometrische Titration nach nach der in den Beispielen beschriebenen Methode.

Da die Bestimmung der mittleren Oxidationsstufe des Vanadiums während der Kalzinierung aus apparativen und zeitlichen Gründen nur äußerst schwierig zu bestimmen ist, ist der erforderliche Zeitraum vorteilhafterweise in Vorversuchen experimentell zu bestimmen. In der Regel dient hierzu eine Meßreihe, bei der unter definierten Bedingungen getempert wird, wobei die Proben nach unterschiedlichen Zeiten aus dem System entfernt, abgekühlt und bezüglich der mittleren Oxidationsstufe des Vanadiums analysiert werden.

Der bei Schritt (a) erforderliche Zeitraum ist im Allgemeinen abhängig von der Natur des Katalysatorprecursors, der eingestellten Temperatur und der gewählten Gasatmosphäre, insbesondere des Sauerstoff-Gehalts. Im Allgemeinen erstreckt sich der Zeitraum bei Schritt (a) auf eine Dauer von über 0,5 Stunden und bevorzugt von über 1 Stunde. Im Allgemeinen ist ein Zeitraum von bis zu 4 Stunden, bevorzugt von bis zu 2 Stunde zur Einstellung der gewünschten mittleren Oxidationsstufe ausreichend. Unter entsprechend eingestellten Bedingungen (z.B. unterer Bereich des Temperaturintervalls und/oder geringer Gehalt an molekularem Sauerstoff) kann aber auch ein Zeitraum von über 6 Stunden erforderlich sein.

Bei Schritt (b) wird die erhaltene Katalysatorzwischenstufe in einer nicht-oxidierenden Atmosphäre mit einem Gehalt an molekularem Sauerstoff von $\leq 0,5$ Vol. -% und an Wasserstoffoxid (Wasserdampf) von 20 bis 75 Vol.-%, bevorzugt von 30 bis 60 Vol.-% bei einer Temperatur von 300 bis 500°C und bevorzugt von 350 bis 450°C über einen Zeitraum von $\geq 0,5$ Stunden, bevorzugt 2 bis 10 Stunden und besonders bevorzugt 2 bis 4 Stunden belassen . Die nicht-oxidierende Atmosphäre enthält neben dem genannten Wasserstoffoxid im Allgemeinen überwiegend Stickstoff und/oder Edelgase, wie beispielsweise Argon, wobei hierunter keine Einschränkung zu verstehen ist. Auch Gase, wie beispielsweise Kohlendioxid sind prinzipiell geeignet. Bevorzugt enthält die nicht-oxidierende Atmosphäre $\geq 40$ Vol.-% Stickstoff. Aus der Sicht des durch die Kalzinierungszone(n) geführten Katalysatorprecursors kann die Temperatur während des Kalzinierschrittes (b) konstant gehalten werden, im Mittel steigen oder fallen. Wird Schritt (b) bei einer höheren oder tieferen Temperatur als Schritt (a) durchgeführt, so befindet sich zwischen den Schritten (a) und (b) in der Regel eine Aufheiz- oder Abkühlphase, welche gegebenenfalls in einer weiteren Kalzinierungszone implementiert ist. Um eine verbesserte Trennung zur sauerstoffhaltigen Atmosphäre des Schrittes (a) zu ermöglichen, kann diese weitere Kalzinierungszone zwischen (a) und (b) beispielsweise zux Spülung mit Inertgas, wie beispiels-weise Stickstoff, gespült werden. Bevorzugt wird Schritt (b) bei einer um 50 bis 150 höheren Temperatur als Schritt (a) durchgeführt.

Im Allgemeinen umfasst die Kalzinierung einen weiteren, zeitlich nach Schritt (b) durchzuführenden Schritt (c), bei dem man den kalzinierten Katalysatorprecursor in einer Inertgas-Atmosphäre auf eine Temperatur von $\leq 300°C$, bevorzugt von $\leq 200°C$ und besonders bevorzugt von $\leq 150°C$ abkühlt.

Vor, zwischen und/oder nach den Schritten (a) und (b), beziehungsweise (a), (b) und (c) sind bei der Kalzinierung nach dem erfindungsgemäßen Verfahren weitere Schritte möglich. Ohne limitierend zu wirken seien als weitere Schritte beispielsweise Änderungen in der Temperatur (Aufheizen, Abkühlen), Änderungen in der Gasatmosphäre (Umstellung der Gasatmosphäre), weitere Haltezeiten, Überführungen der Katalysatorzwischenstufe in andere Apparate oder Unterbrechungen des gesamten Kalziniervorgangs genannt.

Da der Katalysatorprecursor in der Regel vor Beginn der Kalzinierung eine Temperatur von < 100°C besitzt, ist dieser vor Schritt (a) üblicherweise aufzuheizen. Das Aufheizen kann unter Anwendung verschiedener Gasatmosphären durchgeführt werden. Vorzugsweise wird das Aufheizen in einer oxidierend wirkenden Atmosphäre, wie unter Schritt (a) definiert, oder einer Inertgas-Atmosphäre, wie unter Schritt (c) definiert, durchgeführt. Auch ein Wechsel der Gasatmosphäre während der Aufheizphase ist möglich. Besonders bevorzugt ist das Aufheizen in der oxidierend wirkenden Atmosphäre, welche auch in Schritt (a) angewendet wird.

Die beim erfindungsgemäßen Verfahren bevorzugt einzusetzenden Katalysatoren weisen ein Phosphor/Vanadium-Atomverhältnis von 0,9 bis 1,5, besonders bevorzugt von 0,9 bis 1,2 und ganz besonders bevorzugt von 1,0 bis 1,1, eine mittlere Oxidationsstufe des Vanadiums von +3 , 9 bis +4, 4 und besonders bevorzugt von 4,0 bis 4,3, eine BET-Oberfläche von 10 bis 50 m$^2$/g und besonders bevorzugt von 20 bis 40 m$^2$/g, ein Porenvolumen von 0,1 bis 0,5 ml/g und besonders bevorzugt von 0, 2 bis 0,4 ml/g und eine Schüttdichte von 0,5 bis 1,5 kg/l und besonders bevorzugt 0,5 bis 1,0 kg/l auf.

Als Kohlenwasserstoffe mit mindestens vier Kohlenstoffatomen können beim erfindungsgemäßen Verfahren aliphatische und aromatische, gesättigte und ungesättigte Kohlenwasserstoffe mit mindestens vier Kohlenstoffatomen, wie beispielsweise 1,3-Butadien, 1-Buten, 2-cis-Buten, 2-trans-Buten, n-B-utan, $C_4$-Gemisch, 1,3-Pentadien, 1,4-Pentadien, 1-Penten, 2-cis-Penten, 2-trans-Penten, n-Pentan, Cyclopentadien, Dicyclopentadien, Cyclopenten, Cyclopentan, $C_5$-Gemisch, Hexene, Hexane, Cyclohexan und Benzol geeignet. Bevorzugt eingesetzt werden 1-Buten, 2-cis-Buten, 2-trans-Buten, n-Butan, Benzol oder deren Mischungen. Besonders bevorzugt ist der Einsatz von n-Butan und n-Butan-haltigen Gasen und Flüssigkeiten. Das verwendete n-Butan stammt dabei bevorzugt aus dem Erdgas, aus Steamcrackern oder FCC-Crackern.

Die Zugabe des Kohlenwasserstoffs erfolgt im Allgemeinen mengengeregelt, d.h. unter stetiger Vorgabe einer definierten Menge pro Zeiteinheit. Der Kohlenwasserstoff kann in flüssiger oder gasförmiger Form dosiert werden. Bevorzugt ist die Dosierung in flüssiger Form mit anschließender Verdampfung vor Eintritt in dem Rohrbündelreaktor .

Als Oxidationsmittel werden Sauerstoff enthaltende Gase, wie beispielsweise Luft, synthetische Luft, ein mit Sauerstoff angereichertes Gas oder auch sogenannter "reiner", d.h. z.B. aus der Luftzerlegung stammender Sauerstoff eingesetzt. Auch das Sauerstoff-enthaltende Gas wird mengengeregelt zugegeben.

Das durch den Rohrbündelreaktor zu leitende Gas enthält im Allgemeinen eine Kohlenwasserstoff-Konzentratäon von 0,5 bis 15 Vol.-% und eine Sauerstoff-Konzentration von 8 bis 25 Vol.-%. Der zu einhundert Vol.-% fehlende Anteil setzt sich aus weiteren Gasen wie beispielsweise Stickstoff, Edelgasen, Kohlenmonoxid, Kohlendioxid, Wasserdampf, oxygenierte Kohlenwasserstoffe (z.B. Methanol, Formaldehyd, Ameisensäure, Ethanol, Acetyaldehyd, Essigsäure, Propanol, Propionaldehyd, Propionsäure, Acrolein, Crotonaldehyd) und deren Mischungen zusammen. Bevorzugt beträgt der n-Butan-Anteil an der Gesamtmenge an Kohlenwasserstoff ≥90% und besonders bevorzugt ≥95%.

Zur Gewährung einer langen Katalysatorstandzeit und weiterer Erhöhung von Umsatz, Selektivität, Ausbeute, Katalysator-Belastung und Raum/Zeit-Ausbeute wird dem Gas beim erfindungsgemäßen Verfahren bevorzugt eine flüchtige Phosphorverbindung zugeführt. Ihre Konzentration beträgt im Feed am Reaktoreingang mindestens 0,2 Volumen-ppm, d.h. $0,2.10^{-6}$ Volumenanteile der flüchtigen Phosphorverbindungen bezogen auf das Gesamtvolumen des Gases am Reaktoreingang. Bevorzugt ist ein Gehalt von 0,2 bis 20 VoLumen-ppm, besonders bevorzugt von 0,5 bis 10 Volumen-ppm. Als flüchtige Phosphorverbindungen sind all jene Phosphor-enthaltende Verbindungen zu verstehen, welche in der gewünschten Konzentration unter den Einsatzbedingungen gasförmig vorliegen. Als geeignete flüchtige Phosphorverbindungen sind beispielsweise Phosphine und Phosphorsäureester genannt. Besonders bevorzugt ist Tri-($C_1$- bis $C_4$-alkyl)-phosphat und ganz besonders bevorzugt Trimethylphosphat, Triethylphosphat und Tripropylphosphat, insbesondere Triethylphosphat.

Das erfindungsgemäße Verfahren wird bei einer Temperatur von 350 bis 500°C durchgeführt. Unter der genannten Temperatur ist die mittlere Temperatur des Wärmeträgermediums zu verstehen. Bevorzugt wird das erfindungsgemäße Verfahren bei einer Temperatur von 380 bis 460°C und besonders bevorzugt 380 bis 440°C durchgeführt.

Das erfindungsgemäße Verfahren kann bei einem Druck unterhalb von Normaldruck (z.B. bis 0,05 MPa abs) als auch oberhalb von Normaldruck (z.B. bis 10 MPa abs) ausgeübt werden. Darunter ist der in der Rohrbündelreaktor-Einheit am Reaktorausgang vorliegende Druck zu verstehen. Bevorzugt ist ein Druck von 0,075 bis 1,0 MPa abs, besonders bevorzugt 0,075 bis 0,5 MPa abs.

Das erfindungsgemäße Verfahren kann in zwei bevorzugten Verfahrensvarianten, der Variante mit "geradem Durchgang" und der Variante mit "Rückführung" durchgeführt werden. Beim "geraden Durchgang" wird aus dem Reaktoraustrag Maleinsäureanhydrid und gegebenenfalls oxygenierte Kohlenwasserstoff-Nebenprodukte entfernt und das verbleibende Gasgemisch ausgeschleust und gegebenenfalls thermisch verwertet. Bei der "Rückführung" wird aus dem Reaktoraustrag ebenfalls Maleinsäureanhydrid und gegebenenfalls oxygenierte Kohlenwasserstoff-Nebenprodukte entfernt, das verbleibende Gasgemisch, welches nicht-umgesetzten Kohlenwasserstoff enthält, ganz oder teilweise zum Reaktor rückgeführt. Eine weitere Variante der "Rückführung" ist die Entfernung des nicht-umgesetzten Kohlenwasserstoffs und dessen Rückführung zum Reaktor.

In einer besonders bevorzugten Ausf-ührungsform zur Herstellung von Maleinsäureanhydrid setzt man n-Butan als Ausgangs-Kohlenwasserstoff ein und führt die heterogenkatalytische Gasphasenoxidation in Gegenwart von Luft als Sauerstoff enthaltendes Gas und von Triethylphosphat als flüchtige Phosphorverbindung in einer Rohrbündelreaktor-Einheit mit zwei, durch jeweils einen Salzschmelze-Kreislauf gekühlten Reaktionszonen im "geraden Durchgang" durch. Die erste Reaktioxiszone wird dabei in einem nach den Formeln (I) und (II) ermittelten Bereich und die zweite Reaktionszone in einem nach den Formeln (III) und (IV) ermittelten Bereich betrieben. Die dafür erforderlichen Werte für $T_D$ (1. Zone), $T_{SB,Amax}$ (1. Zone), $T_D$(2. Zone) und $T_{SB,Amax}$ (2. Zone) wurden dabei in vorhergehenden Versuchen in einer geeigneten Versuchsanlage experimentell ermittelt.

Das erfindungsgemäße Verfahren zur Herstellung von Maleinsäureanhydrid ermöglicht eine in Bezug auf das Durchgehen der Reaktion sicherheitstechnisch unproblematische Reaktionsführung und führt auch bei hoher Kohlenwasserstoff-Belastung des Katalysators über einen langen Zeitraum von mehreren Monaten bis einigen Jahren hinweg zu einem hohen Umsatz, einer hoher Selektivität sowie einer hohen Ausbeute an Wertprodukt. Dadurch wird eine hohe Raum-Zeit-Ausbeute erzielt und dennoch eine frühzeitige Schädigung des Katalysators vermieden oder zumiridest stark gemindert.

Definitionen

Die in dieser Schrift verwendeten Größen sind, falls nicht anders erwähnt, wie folgt definiert:

$$\text{Raum/Zeit-Ausbeute} = \frac{m_{Maleinsäureanhydrid}}{V_{Katalysator} \cdot t}$$

$$\text{Belastung} = \frac{V_{Kohlenwasserstoff}}{V_{Katalysator} \cdot t}$$

$$\text{GHSV (gaseous hourly space velocity)} = \frac{V_{Gas}}{V_{Katalysator} \cdot t}$$

$$\text{Umsatz } U = \frac{n_{KW,Reaktor,ein} - n_{KW,Reaktor,aus}}{n_{KW,Reaktor,ein}}$$

$$\text{Selektivität } S = \frac{n_{MSA\,Reaktor,aus}}{n_{KW,Reaktor,ein} - n_{KW,Reaktor,aus}}$$

$$\text{Ausbeute } A = U \cdot S$$

| | |
|---|---|
| $m_{Maleinsaureanhydrid}$ | Masse an produziertem Maleinsäureanhydrid [g] |
| $V_{Katalysator}$ | Schüttvolumern Katalysator, summiert über alle Reaktionszonen [1] |
| $t$ | Zeiteinheit [h] |
| $V_{Kohlenwasserstoff}$ | auf 0°C und 0,1013 MPa normiert es Volumen des Kohlenwasserstoffs in der Gasphase am Reaktoreingang [N1] (Rechnerische Größe. Liegt ein Kohlenwasserstoff unter diesen Bedingungen in der Flüssigphase vor, so wird über das ideale Gasgesetz das hypothetische Gasvolumen berechnet.) |
| $V_{Gas}$ | auf 0°C und 0 1013 MPa normiertes Volumen der gesamten Gasmenge am Reaktoreingang [Nl] |
| $U$ | Umsatz an Kohlenwasserstoffen pro Reaktordurchgang |
| $S$ | Selektivität bzgl. Maleinsäureanhydrid pro Reaktordurchgang |
| $A$ | Ausbeute an Maleinsäureanhydrid pro Reaktordurchgang |
| $n_{KW}$, Reaktor, ein | Stoffmengenstrom an Kohlenwasserstoffen am Reaktoreingang [mol/h] |
| $n_{KW}$, Reaktor, aus | Stoffmengenstrom an Kohlenwasserstoffen am Reaktorausgang [mol/h] |
| $n_{MSA}$, Reaktor, aus | Stoffmengenstrom an Maleinsäureanhydrid am Reaktorausgang [mol/h] |
| $T_{SB}$(1. Zone): | Mittlere Salzbad-Temperatur in der ersten Reaktionszone |

$T_{HS}$(1. Zone):            Heißpunkt-Temperatur in der ersten Reaktionszone

$T_{SB}$(2. Zone):            Mittlere Salzbad-Temperatur in der zweiten Reaktionszone

$T_{HS}$(2. Zone):            Heißpunkt-Temperatur in der zweiten Reaktionszone

Beispiele

Bestimmung der mittleren Oxidationsstufe des Vanadiums

Die Bestimmung der mittleren Oxidationsstufe des Vanadiums erfolgte über potentiometrische Titration. Zur Bestimmung werden jeweils 200 bis 300 mg der Probe unter Argonatmosphäre in eine Mischung aus 15 mL 50%-iger Schwefelsäure und 5 mL 85%-iger Phosphorsäure gegeben und unter Erwärmen gelöst. Die Lösung wird anschließend in ein Titrationsgefäß, welches mit zwei Pt-Elektroden ausgestattet ist, überführt. Die Titrationen werden jeweils bei 80°C durchgeführt. Zuerst erfolgt eine Titration mit 0,1 molarer Kaliumpermanganat-Lösung. Werden zwei Stufen in der potentiornetrischen Kurve erhalten, so lag das Vanadium in einer mittleren Oxidationsstufe von +3 bis kleiner +4 vor. Wird nur eine Stufe erhalten, so lag das Vanadium in einer Oxidationsstufe von +4 bis kleiner +5 vor.
Bei dem erstgenannten Fall (zwei Stufen / $+3 \leq V_{ox} < +4$) enthält die Lösung kein $V^{5+}$, das heißt das gesamte Vanadium wurde titrimetrisch erfaßt. Über den Verbrauch der 0,1 molaren Kaliumpermanganat-Lösung und der Lage der beiden Stufen wird die Menge an $V^{3+}$ und $V^{4+}$ berechnet. Der gewichtete Mittelwert ergibt dann die mittlere Oxidationsstufe.
Bei dem zweitgenannten Fall (eine Stufe / $+4 \leq V_{ox} < +5$) kann aus dem Verbrauch der 0,1 molaren Kaliumpermangan at-Lösung die Menge an $V^{4+}$ berechnet werden. Durch anschließende Reduktion des gesamten $V^{5+}$ der erhaltenen Lösung mit einer 0,1 molaren Ammonium-eisen(II)-sulfat-Lösung und erneute Oxidation mit 0,1 molarer Kaliumpermanganat-Lösung kann die Gesamtmenge an Vanadium berechnet werden. Die Differenz zwischen der Gesamtmenge an Vanadium und der Menge an $V^{4+}$ ergibt die ursprünglich vorhandene Menge an $V^{5+}$. Der gewichtete Mittelwert ergibt dann die mittlere Oxidationsstufe.

Bestimmung der Seitendruckfestigkeit der Hohlzylinder

Zur Bestimmung der Seitendruckfestigkeit wurden in nacheinanderfolgenden Messungen die Hohlzylinder mit der gerundeten Seitenfläche jeweils auf die plane Metall-Auflageplatte einer entsprechenden Meßeinrichtung gestellt. Die beiden planparallelen Dekkelflächen befanden sich somit in vertikaler Richtung. Nun wurde ein planer Metall-Stempel von oben mit einer Vorschubgeschwindig-keit von 1,6 mm/min auf den Hohlzylinder zugefahren und der zeitliche Verlauf der Krafteinwirkung auf den Hohlzylinder bis zu dessen Bruch aufgezeichnet. Die Seitendruckfestigkeit des einzelnen Hohlzylinders entspricht der maximal eingewirkten Kraft.
Zur Bestimmung der Seitendruckfestigkeit wurden unter Mittelwertbildung jeweils 30 Einzelmessungen durchgeführt.

Bestimmung des Abriebs

Zur Bestimmung des Abriebs wurden etwa 50 g entstaubter Hohlzylinder in eine Plexiglastrommel mit einem Innendurchmesser von 290 mm, einer Trommelhöhe von 40 mm und einem zwischen Drehachse und Außenwandung befindlichen, mit der Plexiglastrommel fest verbundenen, kreisförmig gekrümmten (Radius 80 mm) Plexiglaseinbau, welcher die gesamte Trommelhöhe von 40 mm umfasst, gegeben. Nun wurde die Plexiglastrommel, deren Drehachse sich in horizontaler Richtung befand, 18 Minuten lang mit 25 Umdrehungen pro Minute gedreht. Anschließend wurde der Abrieb der Probe abgesiebt, die verbliebenen Partikel entstaubt und zurückgewogen. Der Abriebswert ergibts sich nun aus dem Quotienten der Verlustmasse zur ursprünglich eingewogenen Masse.

Versuchsanlage

Die Versuchsanlage war ausgestattet mit einer Zufuhr-Einheit und einem Reaktorrohr. Der Ersatz eines Rohrbü-ndelreaktors durch ein Reaktorrohr ist im Labor- oder Technikumsmaßstab sehr gut möglich, sofern die Abmessungen des Reaktorrohres im Bereich eines technischen Reaktorrohres liegen. Die Anlage wurde im "geraden Durchgang" betrieben.
Der Kohlenwasserstoff wurde mengengeregelt in flüssiger Form über eine Pumpe zugegeben. Als Sauerstoffhaltiges Gas wurde Luft mengengeregelt zugegeben. Triethylphosphat (TEP) wurde ebenfalls mengengeregelt, gelöst in Wasser, in flüssiger Form zugegeben.

Die Rohrbündelreaktor-Einheit bestand aus einem Rohrbündelreaktor mit einem Reaktorrohr. Die Länge des Reaktorrohrs betrug 6,5 m, der Innendurchmesser 22, 3 mm. Innerhalb des Reaktorrohres befand sich in einem Schutzrohr mit 6 mm Außendurchmesser ein Multi-Thermoelement mit 20 Temperaturmeßstellen. Die Temperierung des Reaktors erfolgte durch zwei getrennt regesbare, hintereinanderliegende Wärmeträger-Kreisläufe mit einer Länge von jeweils 3,25 m. Als Wärmeträgermedium wurde eine Salzschmelze eingesetzt.

Das Reaktorrohr wurde von dem Reaktionsgasgemisch von oben nach unten durchströmt. Die oberen 0,2 m des 6,5 m langen Reaktorrohres blieben ungefüllt. Als Nächstes folgte eine 0,3 m lange Vorheizzone, welche mit Steatitformkörpern als Inertmaterial gefüllt war. Im Anschluß an die Vorheizzone folgte die Katalysatorschüttung, welche insgesamt 2144 mL Katalysator enthielt.

Direkt nach der Rohrbündelreaktor-Einheit wurde gasförmiges Produkt entnommen und der gaschromatographischen on-line Analytik zugeführt. Der Hauptstrom des gasförmigen Reaktoraustrags wurde aus der Anlage ausgeschleust.

Herstellung des Katalysators

Im einem mit Stickstoff inertisierten, über Druckwasser außenbeheizbaren 8 m$^3$-Stahl/Email-Rührkessel mit Strombrechern wurden 6,1 m$^3$ Isobutanol vorgelegt. Nach Inbetriebnahme des dreistufigen Impellerrührers wurde das Isobutanol unter Rückfluß auf 90°C aufgeheizt. Bei dieser Temperatur wurde nun über die Förderschnecke mit der Zugabe von 736 kg Vanadiumpentoxid begonnen. Nachdem nach ca. 20 Minuten etwa 2/3 der gewünschten Menge an Vanadi umpentoxid zugegeben wurden, wurde bei weiterer Zugabe an Vanadiunpentoxid mit der Einpumpung von 900 kg 105%-iger Phosphorsäure begonnen. Zur Reinigung der Pumpe wurden weitere 0,2 m$^3$ Isobutanol nachge-pumpt. Anschließend wurde das Reaktionsgemisch unter Rückfluß auf etwa 100 bis 108°C erhitzt und unter diesen Bedingungen 14 Stunden belassen. Im Anschluß daran wurde die heiße Suspension in eine zuvor mit Stickstoff inertisierte und beheizte Druckfil ternutsche abgelassen und bei einer Temperatur von etwa 100°C bei einem Druck oberhalb der Filternutsche von bis zu 0,35 MPa abs abfiltriert. Der Nutschkucken wurde durch stetiges Einleiten von Stickstoff bei 100°C und unter Rühren mit einem mittig angeordnetern, in der Höhe verstellbaren Rührer innerhalb von etwa einer Stunde trockengeblasen. Nach dem Trockenblasen wurde auf ca. 155°C aufgeheizt und auf einen Druck von 15 kPa abs (150 mbar abs) evakuiert. Die Trocknung wurde bis zu einem Rest-Isobutanosgehalt von < 2 Gew.-% im getrockneten Katalysator-Precursor durchgeführt.

Anschließend wurde das getrocknete Pulver 2 Stunden unter Luft in einem Drehrohr mit einer Länge von 6,5 m, einem Innendurchmesser von 0,9 m und innenliegenden spiralförmigen Wendeln behandelt. Die Drehzahl des Drehrohres betrug 0,4 U/min. Das Pulver wurde in einer Menge von 60 kg/h in das Drehrohr gefördert. Die Luftzufuhr betrug 100 m$^3$/h. Die direkt an der Außenseite des Drehrohrs gemessenen Temperaturen der fünf gleichlangen Heizzonen betrugen 250°C, 300°C, 340°C, 340°C und 340°C. Nach dem Abkühlen auf Raumtemperatur wurde der VPO-Precursor mit 1 Gew.-% Graphit innig vermischt und in einem Walzen-Kompaktor kompaktiert. Das Feingut im Kompaktat mit einer Partikelgröße < 400 µm wurde abgesiebt und erneut der Kompaktierung zugeführt. Das Grobgut mit einer Part ikelgröße ≥ 400 µm wurde mit weiteren 2 Gew.-% Graphit vermischt und in einer Tablettiermaschine zu 5x3×2,5 mm Hohlzylindern (äußerer Durchmesser x Höhe x Durchmesser des inneren Lochs) mit einer Seitendruckfestigkeit von 11 N tablettiert. Um die erforderliche Menge an Katalysatorprecursor zu erhalten, wurden mehrere Ansätze durchgeführt.

Etwa 2,7 t der erhaltenen 5×3×2,5 mm Hohlzylindern wurden in einer Schütthöhe von 9 bis 10 cm kontinuierlich auf ein gasdurchlässiges Förderband einer Bandkalziniervorrichtung aus zwei hintereinandergeschalteten, identischen Bandkalzinierapparaten mit insgesamt acht Kalzinierzonen gegeben. Die ersten 1,4 t wurden zur einmaligen Einstellung der Betriebsparameter der Bandkalziniervorrichtung verwendet. Da sie kein einheitliches Material darstellten, wurden sie im Folgenden nicht weiter betrachtet.

Die Bandkalziniervorrichtung wurde bei Atmosphärendruck betrieben. Zwischen den Kalzinierzonen 4 und 5 befand sich eine umkapselte Übergangszone. Jede der acht Kalzinierzonen umfasste zur Erzeugung einer Gaszirkulation jeweils einen Ventilator. Jede der acht Kalzinierzonen wurde mit der gewünschten Menge an gewünschtem Frischgas versorgt. Zur Erhaltung des gewünschten Atmosphärendrucks wurde eine entsprechencle Gasmenge abgeführt. Das Volumen des pro Zeiteinheit in jeder Kalzinierungszone zirkulierenden Gases war größer als das Volumen des pro Zeiteinheit zu- oder abgeführten Gases. Zwischen zwei au feinanderfolgenden Kalzinierungszonen befand sich zur Verringerung des Gasaustausches jeweils eine Trennwand, welche im Bereich des Stromes des Katalysatorprecursors offen war. Die Länge jeder Kalzinierzone betrug 1,45 m. Die Geschwindigkeit des Förderbandes wurde entsprechend der gewünschten Verweilzeit von etwa 2 Stunden pro Kalzinierzone eingestellt. Die einzelnen Zonen wurden wie in Tabelle 1 dargestellt betrieben :

Tabelle 1:

| Parameter zum Betrieb der Bandkal ziniervorrichtung. | | |
|---|---|---|
| Zone | Temperatur | zugeführtes Frischgas |
| Kafzinierzone 1 | Aufheizen auf 250°C | Luft |
| Kalzinierzone 2 | Halten bei 250°C | Luft |
| Kalzinierzone 3 | Halten bei 250°C | Luft |
| Kalzinierzone 4 | Aufheizen auf 310°C | Luft |
| Ubergangszone | Abkühlen auf 200°C | Luft |
| Kalzinierzone 5 | Aufheizen auf 425 °C | $N_2$ |
| Kalzinierzone 6 | Halten bei 425°C | $N_2/H_2O$-Dampf (1:1) |
| Kalzinierzone 7 | Halten bei 425°C | $N_2/H_2O$-Dampf (1:1) |
| Kalzinierzone 8 | Abkühlen auf Raumtemperatur | $N_2$ |

Auf diese Weise wurden ca. 1,3 t fertiger Katalysator kontinuierlich hergestellt. Eine repräsentative Durchschnittsprobe dieses Katalysators wies folgende Daten auf:

*   mittlere Oxidationsstufe des Vanadiums ($V_{ox}$): 4,16
*   Seitendruckfestigkeit (SDF) : 10,1 N
*   Abrieb: 0 ,7 Gew.-%.

Beispiel 1: Einzonenreaktor (2,0 Vol.-% n-Butan)

Beim Einzonenreaktor wurden beide Wärmeträger-Kreisläufe bei gleicher Salzbad-Temperatur betrieben. Die eingestellten Reaktionsbedingungen waren wie folgt:

| Gesamtmenge an eingebautem Katalysator | 2144 mL |
|---|---|
| Konzentration an n-Butan am Reaktoreingang | 2,0 Vol.-% |
| GHSV | 2000 Nl/l$_{Katalysator}$ · h |
| Konzentration an Triethylphosphat (TEP) am Reaktoreingang | 2 Vol.-ppm |
| Konzentration an Wasserdampf am Reaktoreingang | 3 Vol.-% |

Beispiel 1A: Bestimmung der Durchgehtemperatur $T_D$ (1. Zone)

Der Katalysator wurde bei einer mittleren Salzbad-Temperatur $T_{SB}$ von 360°C, einer Konzentration an n-Butan am Reaktoreingang von 1,0 Vol.-%, einer GHSV von 1700 Nl/l$_{Katalysator}$ · h und einer Konzentration an Wasserdampf am Reaktoreingang von 3 Vol.-% angefahren. Anschließend wurde innerhalb von 7 Tagen die Konzentration an n-Butan am Reaktoreingang auf 2,0 Vol. -%, die GHSV auf 2000 Nl/l$_{Katalysator}$ h und die mittlere Salzbad-Tenuperatur $T_{SB}$ auf 393°C sukzessive erhöht und unter diesen Bedingungen die Erreichung eines stabilen Betriebszustands, das heißt eines Zustandes, bei dem innerhalb von 24 Stunden die Drift der Heißpunkt-Temperatur $T_{HS}$ (1. Zone) ≤ 0, 5°C betrug, abgewartet. Nun wurde die mittlere Salzbad-Temperatur schrittweise, zunächst um jeweils 2°C, ab 401°C um jeweils 1°C angehoben. Nach Erreichen eines stabilen Betriebszustands wurden jeweils die Heißpunkt-Temperatur $T_{HS}$(1. Zone), der Umsatz U, die Ausbeute A und die Selektivität S bestimmt. Bei den eingestellten Salzbad-Temperaturen befanden sich alle Heißpunkte im Bereich des oberen Wärmeträg-erkreislaufs.

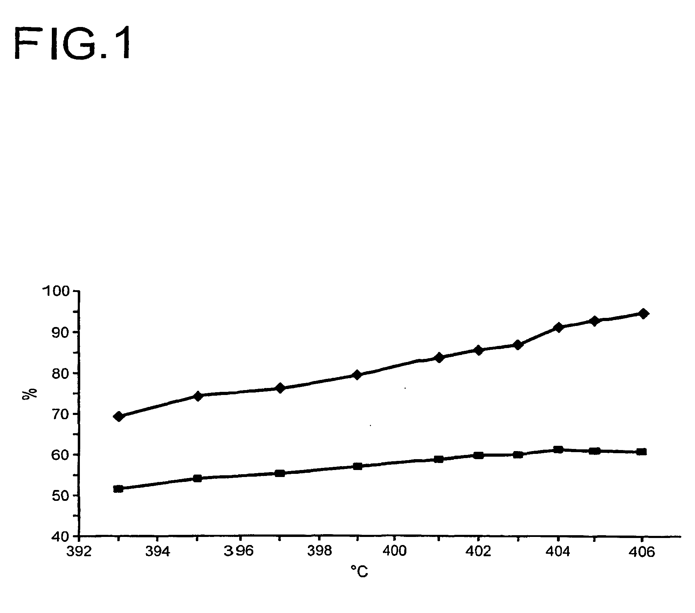

Abbildung 1 zeigt den Verlauf von Umsatz U und Ausbeute A in Abhängigkeit von der mittleren Salzbad-Temperatur. Mit steigender mittlerer Salzbad-Temperatur steigt der Umsatz U in dem untersuchten Bereich kontinuierlich an, wohingegen die Ausbeute an Maleinsäureanhydrid bei einer mittleren Salzbad-Temperatur $T_{SB,Amax}$(1. Zone) von 404°C ein Maximum von 61,1% aufweist.

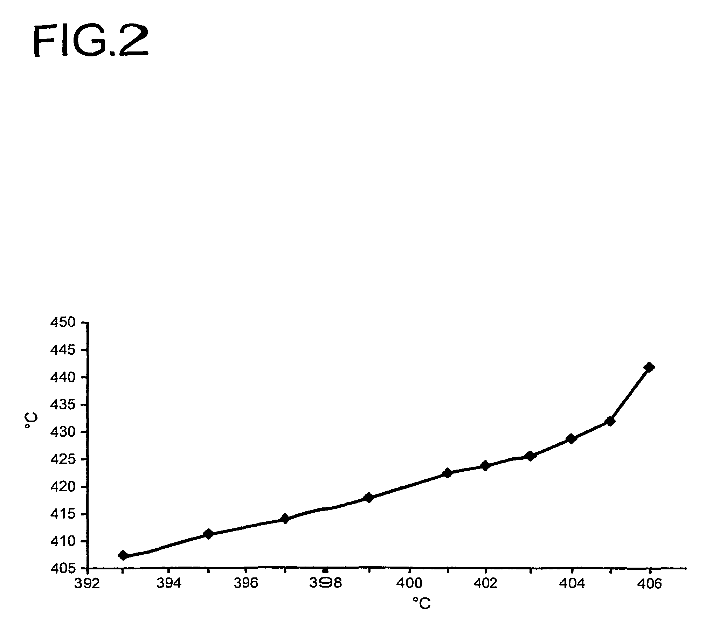

Abbildung 2 gibt die Heißpunkt-Temperatur $T_{HS}$(1. Zone) in Abhängigkeit von der mittleren Salzbad-Temperatur $T_{SB}$(1. Zone) wieder. Eine Erhöhung der mittleren Salzbad-Temperatur $T_{SB}$(1. Zone) von 404°C um 1°C auf 405°C führt zu einer Erhöhung der Heißpunkt-Temperatur $T_{HS}$(1. Zone) um 3,3°C. Eine weitere Erhöhung der mittler en Salzbad-Temperatur $T_{SB}$(1. Zone) um 1°C auf 406°C führt zu einer Erhöhung der Heißpunkt-Temperatur $T_{HS}$ (1. Zone) um 10,0°C. Die Durchgehtemperatur $T_D$(1. Zone) liegt somit zwischen 405 und 406°C. Ei ne näherungsweise Auswertung unter Zugrundelegung der linearen Interpolation zwischen den Meßwerten ergibt eine Durchgehtemperatur $T_D$(1. Zone) von etwa 405,3°C.

Beispiel 1B: Auswahl des Betriebsbereichs des Einzonenreaktors

Gemäß der experimentell ermittelten Durchgehtemperatur $T_D$(1. Zone) von 405,3°C folgt nach Formel (I) ein Bereich für die einzustellende mittlere Salzbad-Temperatur $T_{SB}$(1. Zone) von

$$T_{SB} \text{ (1. Zone)} \leq 405,3°C - 1°C$$

und gemäß der experimentell ermittelten mittleren Salzbad-Temperatur $T_{SB,Amax}$ (1. Zone) von 404°C, bei der das Maximum der Ausbeute an Maleinsäureanhydrid erreicht im Bereich von $T_{SB}$(1. Zone) $\leq$ 405,3°C wurde, nach Formel (II) ein Bereich für die einzustellende mittlere Salzbad-Temperatur $T_{SB}$ (1. Zone) von

$$404°C - 20°C \leq T_{SB}\text{(1. Zone)} \leq 404°C + 10°C.$$

Somit ergibt sich für den erfindungsgemäßen Betrieb des Einzonenreaktor beim Einsatz des gewählten Katalysators und unter den gewählten Betriebsbedingungen eine mittlere Salzbad-Temperatur $T_{SB}$(1. Zone) im Bereich von 384 bis 404, 3°C. Um jedoch eine möglichst hohe Ausbeute an Maleinsäureanhydrid zu erhalten und eine möglichst hohe Sicherheit zu erreichen ist es besonders vorteilhaft, das Verfahren bei einer mittleren Salzbad-Temperatur $T_{SB}$ (1. Zone) im Bereich von (404 - 5)°C bis (405,3 -2)°C, also im Bereich von. 399 bis 403,3°C zu betreiben, was einer Ausbeute an Maleinsäureanhydrid von etwa 56,9 bis 59,8% entspricht. Insbesondere ist es zur Erreichung einer besonders hohen Sicherheit ganz besonders vorteilhaft, das Verfahren bei einer mittleren Salzbad-Temperatur $T_{SB}$ (1. Zone) im Bereich von (404 - 5)°C bis (405, 3 - 4) °C, also im Bereich von 399 bis 401,3°C zu betreiben, was einer Ausbeute an Maleinsäureanhydrid von etwa 56,9 bis 58,7% entspricht .

Es sei betont, dass das experimentell ermittelte Maximum der Maleinsäureanhydrid-Ausbeute von 61,1% bei einer mittleren Salzbad-Temperatur $T_{SB}$ (1. Zone) von 404°C liegt. Im vorliegenden Fall wird durch Formel (I) die einzustellende mittlere Salzbad-Temperatur $T_{SB}$ (1. Zone) auf $\leq$ 404,3°C, bevorzugt auf 403,3°C und besonders bevorzugt auf 401,3°C begrenzt und dadurch die Gefahr plötzlich auftretender, unkontrollierter Temperaturspitzen im Katalysatorbett des Rohrbündelreaktors, welche den Katalysator irreversibel schädigen könnten, signifikant herabgesetzt beziehungsweise ausgeschlossen. Durch die erfindungsgemäße Maßnahme wird somit dieser gefährliche Bereich ausgeschlossen und ein sicherer Betrieb des Verfahrens sichergestellt sowie eine frühzeitige thermische Schädigung des Katalysators vermieden.

Beispiel 2: Zweizonenrealctor (2,0 Vol.-% n-Butan)

Die eingestellten Reaktionsbedingungen waren wie folgt:

| | |
|---|---|
| Gesamtmenge an eingebautem Katalysator | 2144 mL |
| Konzentration an n-Butan am Reaktoreingang | 2,0 Vol.-% |
| GHSV | 2000 N1/l$_{Katalysator}$ · h |
| Konzentration an Triethylphosphat (TEP) am Reaktoreingang | 2 Vol.-ppm |
| Konzentration an Wasserdampf am Reaktoreingang | 3 Vol.-% |

Da die Reaktionsbedingungen in der oberen Reaktionszone des E inzonenreaktors und der ersten Reaktionszone des Zweizonenreakt ors identisch sind, ist auch die Durchgehtemperatur $T_D$ (1. Zone) des Einzonenreaktors identisch mit der Durchgeh temperatur der ersten Reaktionszone $T_D$ (1. Zone) des Zweizonenreaktors . Daher sei bezüglich der Bestimmung der Durchgehtemperatur der ersten Reaktionszone $T_D$(1. Zone) des Zweizonenreaktors sowie der Auswahl des entsprechenden Betriebsbereichs auf Beispiel 1 verwiesen.

Die erste Reaktionszone des Zweizonenreaktors ist somit beim Einsatz des gewählten Katalysators und unter den gewählten Betriebsbedingungen bei einer mittleren Salzbad-Temperatur $T_{SB}$(1. Zone) im Bereich von 384 bis 404, 3°C, besonders vorteilhaft im Bereich von 399 bis 403,3°C und ganz besonders vorteilhaft im Bereich von 399 bis 401, 3°C zu betreiben.

Beispiel 2A: Bestimmung der Durchgehtemperatur $T_D$ (2. Zone)

Im vorliegenden Beispiel wurde die erste Reaktionszone des Zweizonenreaktors bei einer mittleren Salzbad-Temperatur $T_{SB}$ (1. Zone) von 400°C betrieben.

Der Katalysator wurde nun bei einer mittleren Salzbad-Temperatur der ersten Reaktionszone $T_{SB}$(1. Zone) von 400°C und einer mittleren Salzbad-Temperatur der zweit en Reaktionsone $T_{SB}$ (2. Zone) von 402°C in einen stabilen Betriebszustand gefahren. Nun wurde die mittlere Salzbad-Temperatur der zweiten Reaktionsone $T_{SB}$ (2. Zone) schrittweise um jeweils 1°C angehoben und die Erreichung eines stabilen Betriebszustandes , das heißt eines Z ustandes, bei dem innerhalb von 24 Stunden die Drift der Heißpunkt-Temperatur $T_{HS}$ (2. Zone) ≤ 0,5°C betrug, abgewartet. Nach Erreichen eines stabilen Betriebszustands wurden jeweils die Heißpunkt-Temperatur $T_{HS}$ (2. Zone), der Umsatz U, die Ausbeute A und die Selektivität S bestimmt.

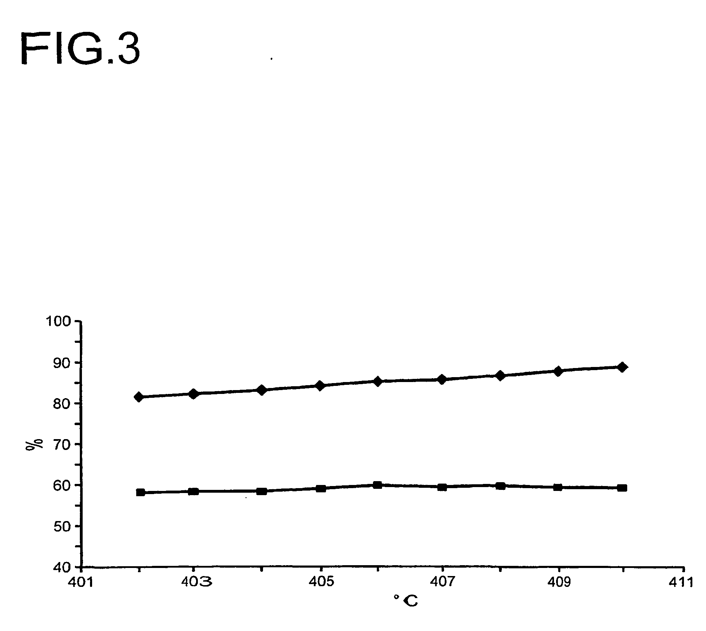

Abbildung 3 zeigt den Verlauf von Umsatz U und Ausbeute A in Abhängigkeit von der mittleren Salzbad-Temperatur der zweiten Reaktionsone $T_{SB}$ (2. Zone). Mit steigender mittlerer Salzbad-Temperatur $T_{SB}$(2. Zone) steigt der Umsatz U in dem untersuchten Bereich kontinuierlich an, wohingegen die Ausbeute an Maleinsäureanhydrid bei einer mittleren Salzbad-Temperatur $T_{SB,Amax}$(2. Zone) von 406 bis 408°C ein Maximum von 59,6% aufweist.

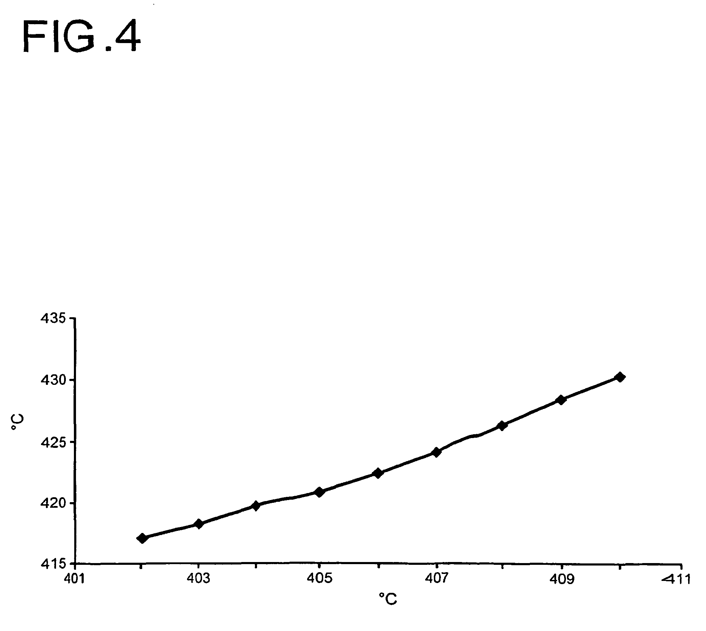

Abbildung 4 gibt die Heißpunkt-Tempera tur der zweiten Reaktionszone $T_{HS}$(2. Zone) in Abhängigkeit von der mittleren Salzbad-Temperatur der zweiten Reaktionszone $T_{SB}$(2. Zone) wieder. Im untersuchten Temperaturbereich von $T_{SB}$(2. Zone) lag die Erhöhung der Heißpunkt-Temperatur $T_{HS}$(2. Zone) bei Erhöhung der mittleren Salzbad-Temperatur $T_{SB}$(2. Zone) um 1°C im Bereich von 1,1 bis 2,2°C. Somit liegt die Durchgehtemperatur der zweiten Reaktionszone $T_D$ (2. Zone) oberhalb von 410°C .

Beispiel 2B: Auswahl des Betriebsbereichs des Zweizonenreaktors

Wie unter Beispiel 2A bereits erwähnt wurde die erste Reaktionszone des Zweizonenreaktors bei einer mittleren Salzbad-Temperatur $T_{SB}$ (1. Zone) von 400°C betrieben.

Da im untersuchten Temperaturbereich von $T_{SB}$(2. Zone) keine Durchgehtemperatur der zweiten Reaktionszone $T_D$(2. Zone) ermittelt werden konnte, ist nach den Ausführungen von Beispiel 2A davon auszugehen, dass diese oberhalb von 410°C liegt. Nach Formel (III) ist somit die mittlere Salzbad-Temperatur TsB(2. Zone) sicherheitshalber auf einen Wert von

$$T_{SB}(2.\ Zone) \leq 410°C - 1°C$$

einzustellen.

Gemäß der experimentell ermittelten mittleren Salzbad-Temperatur $T_{SB,Amax}$ (2. Zone) von 406 bis 408°C (Mittelwert 407°C), bei der das Maximum der Ausbeute an Maleinsäureanhydrid im Bereich von $T_{SB}$(2. Zone) ≤ 410°C erreicht wurde, folgt nach Formel (IV) ein Bereich für die einzustellende mittlere Salzbad-Temperatur $T_{SB}$(2. Zone) von

$$407°C - 10°C \leq T_{SB}(2.\ Zone) \leq 407°C + 10°C.$$

Somit ergibt sich für den erfindungsgemäßen Betrieb des Zweizonenreaktor beim Einsatz des gewählten Katalysators und unter den gewählten Betriebsbedingungen eine mittlere Salzbad-Temperatur $T_{SB}$(2. Zone) im Bereich von 397 bis 409°C. Um jedoch eine möglichst hohe Ausbeute an Maleinsäureanhydrid zu erhalten und eine möglichst hohe Sicherheit zu erreichen ist es besonders vorteilhaft, das Verfahren bei einer mittleren Salzbad-Temperatur $T_{SB}$ (2. Zone) im Bereich von (407 - 5)°C bis (410 - 2)°C, also im Bereich von 402 bis 408°C zu betreiben, was einer Ausbeute an Maleinsäureanhydrid von etwa 58,1 bis 59,6% entspricht. Insbesondere ist es zur Erreichung einer besonders hohen Sicherheit ganz besonders vor teilhaft, das Verfahren bei einer mittleren Salzbad-Temperatur $T_{SB}$(2. Zone) im Bereich von (407 - 5)°C bis (410 - 4)°C, also im Bereich von 402 bis 406°C zu betzeiben, was einer Ausbeute an Maleinsäureanhydrid von etwa 58,1 bis 59,6% entspricht.

Ein Vergleich der Beispiele 1 und 2 zeigt den besonderen Vorteil eines Mehrzonenreaktors, das heißt im vorliegenden Fall eines Zweizonenreaktors. Während beim Einzonenreaktor unter den vorliegenden Bedingungen ein in Bezug auf Ausbeute und Sicherheit ganz besonders vorteilhafter Betrieb bei einer mittleren Salzbad-Temperatur $T_{SB}$(1. Zone) im Bereich von 399 bis 401,3°C möglich ist, was eine Ausbeute an Maleinsäureanhydrid von etwa 56, 9 bis 58,7% ermöglicht, kann beim Einsatz eines Zweizonenreaktors bei einer mittleren Salzbad-Temperatur $T_{SB}$(1. Zone) von 400°C und einer mittleren Salzbad-Temperatur $T_{SB}$(2. Zone) im Bereich von 402 bis 406°C bei vergleichbar hoher Sicherheit eine Ausbeute an Maleinsäureanhydrid von etwa 58,1 bis 59, 6% erreicht werden. Die durch den Einsatz eines Zweizonenreaktors erreichbare Ausbeute liegt somit um etwa 2 rel.-% höher als beim Einsatz eines Einzonenreaktors.

Beispiel 3: Einzonenreaktor (2,2 Vol.-% n-Butan)

Beim Einzonenreaktor wurden beide Wärmeträger-Kreisläufe bei gleicher Salzbad-Temperatur betrieben. Die eingestellten Reaktionsbedingungen waren wie folgt:

| | |
|---|---|
| Gesamtmenge an eingebautem Katalysator | 2144 mL |
| Konzentration an n-Butan am Reaktoreingang | 2,2 Vol.-% |
| GHSV | 2000 Nl/l$_{Katalysator}$·h |
| Konzentration an Triethylphosphat (TEP) am Reaktoreingang | 2 Vol.-ppm |
| Konzentration an Wasserdampf am Reaktoreingang | 3 Vol.-% |

Beispiel 3 wurde im Anschluß an Beispiel 2 mit dem dort verwendeten Katalysator durchgeführt.

Beispiel 3A: Bestimmung der Durchgehtemperatur T$_D$(1. Zone)

Es wurden die oben genannten Reaktionsbedingungen sowie eine mittlere Salzbad-Temperatur T$_{SB}$ von 404°C eingestellt und die Erreichung eines stabilen Betriebszustands, das heißt eines Zustandes, bei dem innerhalb von 24 Stunden die Drift der Heißpunkt-Temperatur T$_{HS}$ (1. Zone) ≤ 0,5°C betrug, abgewartet. Nun wurde die mittlere Salzbad-Temperatur schrittweise um jeweils 1°C angehoben. Nach Erreichen eines stabilen Betriebszustands wurden jeweils die Heißpunkt-Temperatur T$_{HS}$ (1. Zone), der Umsatz U, die Ausbeute A und die Selektivität S bestimmt. Bei den eingestellten Salzbad-Temperaturen befanden sich alle Heißpunkte im Bereich des oberen Wärmeträgerkreislaufs.

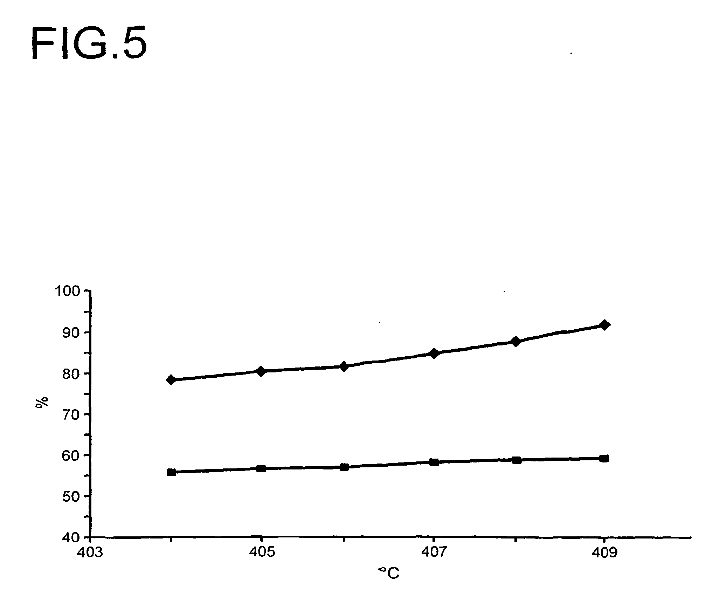

Abbildung 5 zeigt den Verlauf von Umsatz U und Ausbeute A in Abhängigkeit von der mittleren Salzbad-Temperatur. Mit steigender mittlerer Salzbad-Temperatur steigen sowohl der Umsatz U als auch die Ausbeute an Maleinsäureanhydrid in dem untersuchten Bereich kontinuierlich an.

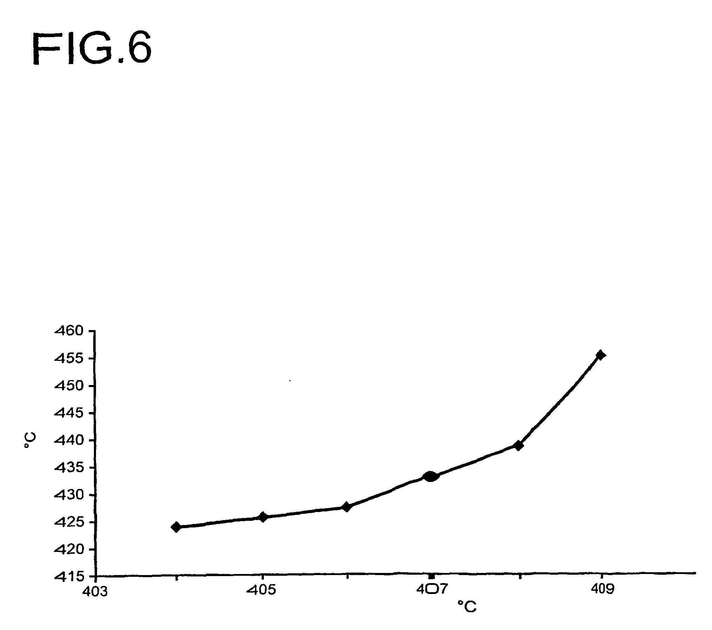

Abbildung 6 gibt die Heißpunkt-Temperatur T$_{HS}$(1. Zone) in Abhängigkeit von der mittleren Salzbad-Temperatur T$_{SB}$ (1. Zone) wieder. Eine Erhöhung der mittleren Salzbad-Temperatur T$_{SB}$ (1. Zone) von 405°C um 1°C auf 406°C führt zu einer Erhöhung der Heißpunkt-Temperatur T$_{HS}$(1. Zone) um 1,8°C. Eine weitere Erhöhung der mittleren Salzbad-Temperatur T$_{SB}$ (1. Zone) um 1°C auf 407°C führt zu einer Erhöhung der Heißpunkt-Temperatur T$_{HS}$(1. Zone) um 5,4°C. Die Durchgehtemperatur T$_D$ (1. Zone) liegt somit zwischen 406 und 407°C. Eine näherungsweise Auswertung unter Zugrundelegung der linearen Interpolation zwischen den Meßwerten ergibt eine Durchgehtemperatur T$_D$ (1. Zone) von etwa 406, 9°C .

Beispiel 3B: Auswahl des Betriebsbereichs des Einzonenreaktors

Gemäß der experimentell ermittelten Durchgeh temperatur T$_D$ (1. Zone) von 406, 9°C ergibt sich nach Formel (I) ein Bereich für die einzustellende mittlere Salzbad-Temperatur T$_{SB}$ (1. Zone) von

$$T_{SB} \text{ (1. Zone)} \leq 406, 9°C - 1°C.$$

Die maximale Ausbeute an Maleinsäureanhydrid wird im Bereich von T$_{SB}$ (1. Zone) ≤ 406,9°C bei 406, 9°C erreicht und beträgt etwa 57,8%. Nach Formel (II) folgt daraus ein Bereich für die einzustellende mittlere Salzbad-Temperatur T$_{SB}$ (1. Zone) von

$$406,9°C - 20°C \leq T_{SB}\text{(1. Zone)} \leq 406,9°C + 10°C.$$

Somit ergibt sich für den erfindungsgemäßen Betrieb des Einzonenreaktor beim Einsatz des gewählten Katalysators und unter den gewählten Betriebsbedingungen eine mittlere salzbad-Temperatur T$_{SB}$(1. Zone) im Bereich von 386,9 bis 405, 9°C . Um jedoch eine möglichst hohe Ausbeute an Maleinsäureanhydrid zu erhalten und eine möglichst hohe Sicherheit zu erreichen ist es besonders vorteilhaft, das Verfahren bei einer mittleren Salzbad-Temperatur T$_{SB}$(1. Zone) im Bereich von (406,9 - 5)°C bis (406,9 -2)°C, also im Bereich von 401,9 bis 404, 9°C zu betreiben, was einer Ausbeute an Maleinsäureanhydrid von bis zu 56,2% entspricht.

Es sei betont, dass das experimentell ermittelte Maximum der Maleinsäureanhydrid-Ausbeute im untersuchten Gesamtbereich von 58,8% bei einer mittleren Salzbad-Temperatur T$_{SB}$(1. Zone) von 409°C und somit deutlich oberhalb der Durchgehtemperatur $T_D$(1. Zone) liegt. Würde das Verfahren entgeg-en der Lehre der vorliegenden Erfindung bei dieser Temperatur betrieben werden, so bestünde die Gefahr plötzlich auftretender, unkontrollierter Temperaturspitzen im Katalysatorbett des Rohrbündelreaktors, welche den Katalysator irreversibel schädigen konnten. Des Weiteren bestünde die Gefahr des "Durchgehens" einzelner Reaktionsrohre bis hin zum gesamten Rohrbündelreaktor.

Durch die erfindungsgemäße Maßnahme wird dieser gefährliche Bereich ausgeschlossen und ein sicherer Betrieb des Verfahrens sichergestellt sowie eine frühzeitige thermische Schädigung des Katalysators vermieden.

Beispiel 4: Zweizonenreaktor (2,2 Vol.-% n-Butan)

Die eingestellten Reaktionsbedingungen waren wie folgt:

| | |
|---|---|
| Gesamtmenge an eingebautem Katalysator | 2144 mL |
| Konzentration an n-Butan am Reaktoreingang | 2, 2 Vol.-% |
| GHSV | 2000 Nl/l$_{Katalysator}$· h |
| Konzentration an Triethylphosphat (TEP) am Reaktoreingang | 2 Vol. -ppm |
| Konzentration an Wasserdampf am Reaktoreingang | 3 Vol. -% |

Da die Reaktionsbedingungen in der oberen Reaktionszone das Einzonenreaktors und der ersten Reaktionszone des Zweizonenreaktors identisch sind, ist auch die Durchgehtemperatur $T_D$(1. Zone) des Einzonenreaktors identisch mit der Durchgehtemperatur der ersten Reaktionszone $T_D$(1. Zone) des Zweizonenreaktors . Daher sei bezüglich der Bestimmung der Durchgehtemperatur der ersten Reaktionszone $T_D$(1. Zone) des Zweizonenreaktors sowie der Auswahl des entsprechenden Betriebsbereichs auf Beispiel 3 verwiesen.

Die erste Reaktionszone des Zweizonenreaktors ist somit beim Einsatz des gewählten Katalysators und unter den gewählten Betriebsbedingungen bei einer mittleren Salzbad-Temperatur $T_{SB}$ (1. Zone) im Bereich von 386,9 bis 405, 9°C und besonders vorteilhaft im Bereich von 401,9 bis 404, 9°C zu betreiben.

Beispiel 4A: Bestimmung der Durchgehtemperatur $T_D$(2. Zone)

Im vorliegenden Beispiel wurde die erste Reaktionszone des Zweizonenreaktors bei einer mittleren Salzbad-Temperatur $T_{SB}$(1 - Zone) von 404°C betrieben.

Der Katalysator wurde nun bei einer mittleren Salzbad-Temperatur der ersten Reaktionszone $T_{SB}$(1. Zone) von 404°C und einer mittleren Salzbad-Temperatur der zweiten Reaktionsone $T_{SB}$ (2. Zone) von 411°C in einen stabilen Betriebszustand gefahren. Nun wurde die mittlere Salzbad-Temperatur der zwei ten Reaktionsone $T_{SB}$(2. Zone) schrittweise um jeweils 1°C angehoben und die Erreichung eines stabilen Betriebszustandes, das heißt eines Zu standes, bei dem innerhalb von 24 Stunden die Drift der Heißpunkt-Temperatur $T_{HS}$ (2. Zone) $\leq 0,5$°C betrug, abgewartet. Nach Erreichen eines stabilen Betriebszustands wurden jeweils die Heißpunkt-Temperatur $T_{HS}$ (2. Zone), der Umsatz U, die Ausbeute A -und die Selektivität S bestimmt.

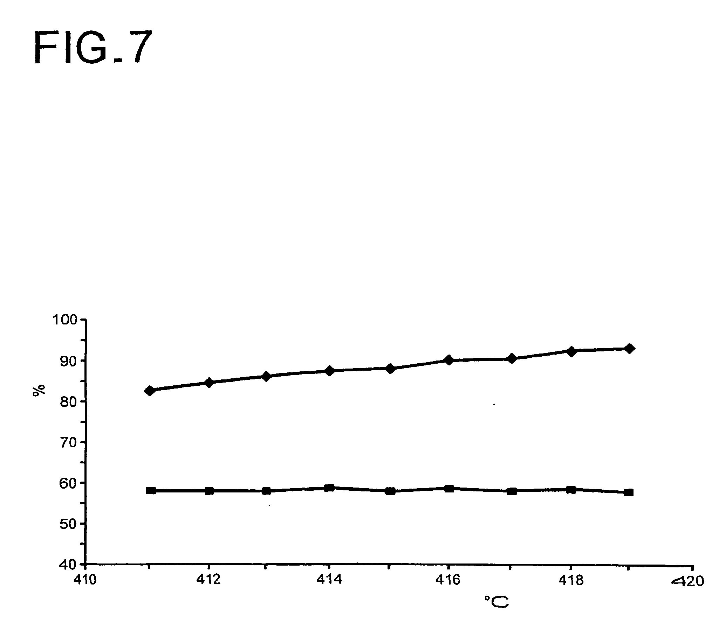

Abbildung 7 zeigt den Verlauf von Umsatz U und Ausbeute A in Abhängigkeit von der mittleren Salzbad-Temperatur der zweiten Reaktionsone $T_{SB}$(2. Zone). Mit steigender mittlerer Salzbad-Temperatur $T_{SB}$(2. Zone) steigt der Umsatz U in dem untersuchten Bereich kontinuierlich an, wohingegen die Ausbeute an Maleinsäureanhydrid bei einer mittleren Salzbad-Temperatur $T_{SB,Amax}$(2. Zone) von 414 bis 418°C ein flaches Maximum von etwa 58,2% aufweist.

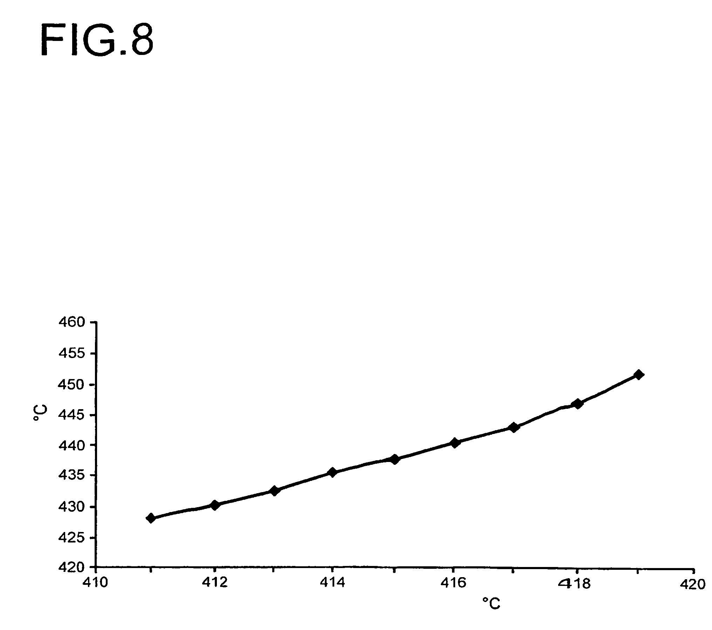

Abbildung 8 gibt die Heißpunkt-Temperatur $T_{HS}$ (2. Zone) in Abhängigkeit von der mittleren Salzbad-Temperatur $T_{SB}$(2. Zone) wieder. Eine Erhöhung der mittleren Salzbad-Temperatur $T_{SB}$ (2. Zone) von 417°C um 1°C auf 418°C führt zu einer Erhöhung der Heißpunkt-Temperatur $T_{HS}$ (2. Zone) um 3, 8°C. Eine weitere Erhöhung der mittleren Salzbad-Temperatur $T_{SB}$ (2. Zone) μm 1°C auf 419°C führt zu einer Erhöhung der Heißpunkt-Temperatur $T_{HS}$ (2. Zone) um 5,1°C. Die Durchgehtemperatur $T_D$ (2 . Zone) liegt somit zwischen 418 und 419°C. Eine näherungsweise Auswertung unter Zugrundelegung der linearen Interpolation zwischen den Meßwerten ergibt eine Durchgehtemperatur $T_D$ (2. Zone) von etwa 418,8°C.

Beispiel 4B: Auswahl des Betriebsbereichs des Zweizonenreaktors Wie unter Beispiel 4A bereits erwähnt wurde die erste Reaktionszone des Zweizonenreaktors bei einer mittleren Salzbad-Temperatur $T_{SB}$(1. Zone) von 404°C betrieben.

Gemäß der experimentell ermittelten Durchgehtemperatur $T_D$(2. Zone) von 418, 8°C ergibt sich nach Formel (III) ein Bereich für die einzustellende mittlere Salzbad-Temperatur $T_{SB}$(2. Zone) von

$$T_{SB}(2.\ Zone) \leq 418{,}8°C - 1°C.$$

Gemäß der experimentell ermittelten mittleren Salzbad-Temperatur $T_{SB,Amax}$ (2. Zone) von 414 bis 418°C (Mittelwert 416°C), bei der das Maximum der Ausbeute an Maleinsäureanhydrid im Bereich von $T_{SB}$(2. Zone) $\leq$ 418,8°C erreicht wurde, folgt nach Formel (IV) ein Bereich für die einzustellende mittlere Salzbad-Temperatur $T_{SB}$ (2. Zone) von

$$416°C - 10°C \leq T_{SB}\ (2.\ Zone) \leq 416°C + 10°C.$$

Somit ergibt sich für den erfindungsgemäßen Betrieb des Zweizonenreaktor beim Einsatz des gewählten Katalysators und unter den gewählten Betriebsbedingungen eine mittlere Salzbad-Temperatur $T_{SB}$ (2. Zone) im Bereich von 406 bis 417, 8°C. Um jedoch eine möglichst hohe Ausbeute an Maleinsäureanhydrid zu erhalten und eine möglichst hohe Sicherheit zu erreichen ist es besonders vorteilhaft, das Verfahren bei einer mittleren Salzbad-Temperatur $T_{SB}$ (2. Zone) im Bereich von (416 - 5)°C bis (418,8 - 2)°C, also im Bereich von 411 bis 416, 8°C zu betreiben, was einer Ausbeute an Maleinsäureanhydrid von etwa 57,7 bis 58,2% entspricht.

Auch ein Vergleich der Beispiele 3 und 4 zeigt den besonderen Vorteil eines Mehrzonenreaktors, das heißt im vorliegenden Fall eines Zweizonenreaktors. Während beim Einzonenreaktor unter den vorliegenden Bedingungen ein in Bezug auf Ausbeute und Sicherheit besonders vorteilhafter Betrieb bei einer mittleren Salzbad-Temperatur $T_{SB}$ (1 - Zone) im Bereich von 401,9 bis 404, 9°C möglich ist, was eine Ausbeute an Maleinsäureanhydrid von bis zu 56,2% ermöglicht, kann beim Einsatz eines Zweizonenreaktors bei einer mittleren Sal zbad-Temperatur $T_{SB}$ (1. Zone) von 404°C und einer mittleren Sal zbad-Temperatur $T_{SB}$ (2. Zone) im Bereich von 411 bis 416, 8°C bei vergleichbar hoher Sicherheit eine Ausbeute an Maleinsäureanhydrid von etwa 57,7 bis 58,2% erreicht werden.

Tabelle 2:

Versuchsdaten zur Ermittlung der Durchgehtemperatur $T_D$(1. Zone) beim Betrieb als Einzonenreaktor (Beispiel 1) sowie der Durchgehtemperatur der ersten Reaktionszone $T_D$(1. Zone) beim Betrieb als Zweizonenreaktor (Beispiel 2)

| $T_{SB}$(1. Zone) [°C] | $T_{HS}$(1. Zone) [°C] | $\Delta T_{HS}$(1. Zone) bei Erhöhung von $T_{SB}$(1. Zone) um 1°C [°C] | Ausbeute A [%] | Selektivität S [%] | Umsatz U [%] |
|---|---|---|---|---|---|
| 393 | 407,2 | 2,1 (interpoliert) | 51,5 | 74,0 | 69,6 |
| 395 | 411,3 | 1,4 (interpoliert) | 54,0 | 72,8 | 74,2 |
| 397 | 414,0 | 1,9 (interpoliert) | 55,2 | 72,5 | 76,2 |
| 399 | 417,7 | 2,3 (interpoliert) | 56,9 | 71,7 | 79,4 |
| 401 | 422,3 | 1,4 | 58,7 | 70,2 | 83,7 |
| 402 | 423,7 | 1,8 | 59,6 | 69,8 | 85,4 |
| 403 | 425,5 | 3,2 | 59,8 | 68,9 | 86,8 |
| 404 | 428,7 | 3,3 | 61,1 | 67,2 | 91,0 |
| 405 | 432,0 | 10,0 | 60,9 | 65,9 | 92,6 |
| 406 | 442,0 | — | 60,7 | 64,2 | 94,6 |

Während der gesamten Versuchsreihe wurden folgende Parameter konstantgehalten:

* Konzentration an n-Butan am Reaktoreingang    = 2,0 Vol.-%
* GHSV    = 2000 Nl/l$_{Katalysator}$ · h
* Druck am Reaktorausgang    = 0,2 MPa abs
* Konzentration an Triethylphosphat (TEP)    = 2 Volumen-ppm
* Konzentration an Wasserdampf    = 3 Vol.-%

EP 1 529 039 B1

## Tabelle 3:

Versuchsdaten zur Ermittlung der Durchgehtemperatur der zweiten Reaktionszone $T_D(2.$ Zone) bei einer mittleren Salzbad-Temperatur der ersten Zone $T_{SB}(1.$ Zone) von 400°C beim Betrieb als Zweizonenreaktor (Beispiel 2)

| $T_{SB}(2.$ Zone) [°C] | $T_{HS}(2.$ Zone) [°C] | $\Delta T_{HS}(2.$ Zone) bei Erhöhung von $T_{SB}(2.$ Zone) um 1°C [°C] | Ausbeute A [%] | Selektivität S [%] | Umsatz U [%] |
|---|---|---|---|---|---|
| 402 | 417,1 | 1,2 | 58,1 | 71,3 | 81,5 |
| 403 | 418,3 | 1,4 | 58,4 | 71,0 | 82,2 |
| 404 | 419,7 | 1,1 | 58,2 | 70,1 | 83,0 |
| 405 | 420,8 | 1,6 | 58,8 | 70,0 | 84,0 |
| 406 | 422,4 | 1,7 | 59,6 | 70,0 | 85,2 |
| 407 | 424,1 | 2,2 | 59,3 | 69,2 | 85,7 |
| 408 | 426,3 | 2,1 | 59,6 | 68,7 | 86,7 |
| 409 | 428,4 | 1,9 | 59,3 | 67,5 | 87,8 |
| 410 | 430,3 | —— | 59,2 | 66,7 | 88,8 |

Während der gesamten Versuchsreihe wurden folgende Parameter konstantgehalten:

* Konzentration an n-Butan am Reaktoreingang     = 2,0 Vol.-%
* GHSV     = 2000 Nl/l$_{Katalysator}$ · h
* Druck am Reaktorausgang     = 0,2 MPa abs
* Konzentration an Triethylphosphat (TEP)     = 2 Volumen-ppm
* Konzentration an Wasserdampf     = 3 Vol.-%

Tabelle 4:

Versuchsdaten zur Ermittlung der Durchgehtemperatur $T_D$(1. Zone) beim Betrieb als Einzonenreaktor (Beispiel 3) sowie der Durchgehtemperatur der ersten Reaktionszone $T_D$(1. Zone) beim Betrieb als Zweizonenreaktor (Beispiel 4)

| $T_{SB}$(1. Zone) [°C] | $T_{HS}$(1. Zone) [°C] | $\Delta T_{HS}$(1. Zone) bei Erhöhung von $T_{SB}$(1. Zone) um 1°C [°C] | Ausbeute A [%] | Selektivität S [%] | Umsatz U [%] |
|---|---|---|---|---|---|
| 404 | 423,8 | 1,7 | 55,4 | 70,8 | 78,3 |
| 405 | 425,5 | 1,8 | 56,2 | 70,3 | 80,0 |
| 406 | 427,3 | 5,4 | 56,5 | 69,4 | 81,4 |
| 407 | 432,7 | 5,9 | 57,8 | 68,5 | 84,4 |
| 408 | 438,6 | 16,4 | 58,5 | 67,0 | 87,3 |
| 409 | 455,0 | — | 58,8 | 64,3 | 91,4 |

Während der gesamten Versuchsreihe wurden folgende Parameter konstantgehalten:

* Konzentration an n-Butan am Reaktoreingang    = 2,2 Vol.-%
* GHSV    = 2000 Nl/l$_{Katalysator}$ · h
* Druck am Reaktorausgang    = 0,2 MPa abs
* Konzentration an Triethylphosphat (TEP)    = 2 Volumen-ppm
* Konzentration an Wasserdampf    = 3 Vol.-%

Tabelle 5:
Versuchsdaten zur Ermittlung der Durchgehtemperatur der zweiten Reaktionszone $T_D$(2. Zone) bei einer

mittleren Salzbad-Temperatur der ersten Zone $T_{SB}$(1. Zone) von 404°C beim Betrieb als Zweizonenreaktor (Beispiel 4)

| $T_{SB}$(2. Zone) [°C] | $T_{HS}$(2. Zone) [°C] | $\Delta T_{HS}$(2. Zone) bei Erhöhung von $T_{SB}$(2. Zone) um 1°C [°C] | Ausbeute A [%] | Selektivität S [%] | Umsatz U [%] |
|---|---|---|---|---|---|
| 411 | 428,1 | 2,0 | 57,7 | 69,8 | 82,7 |
| 412 | 430,1 | 2,4 | 57,7 | 68,4 | 84,4 |
| 413 | 432,5 | 3,0 | 57,7 | 67,2 | 85,9 |
| 414 | 435,5 | 2,1 | 58,3 | 66,9 | 87,1 |
| 415 | 437,6 | 2,7 | 57,8 | 65,8 | 87,8 |
| 416 | 440,3 | 2,7 | 58,2 | 64,7 | 89,9 |
| 417 | 443,0 | 3,8 | 58,0 | 64,2 | 90,3 |
| 418 | 446,8 | 5,1 | 58,2 | 63,1 | 92,2 |
| 419 | 451,9 | — | 57,7 | 62,0 | 93,0 |

Während der gesamten Versuchsreihe wurden folgende Parameter konstantgehalten:

* Konzentration an n-Butan am Reaktoreingang = 2,2 Vol.-%

* GHSV = 2000 Nl/l$_{Katalysator}$ · h

* Druck am Reaktorausgang = 0,2 MPa abs

* Konzentration an Triethylphosphat (TEP) = 2 Volumen-ppm

* Konzentration an Wasserdampf = 3 Vol.-%

EP 1 529 039 B1

**Patentansprüche**

1. Verfahren zur Herstellung von Maleinsäureanhydrid durch heterogenkatalytische Gasphasenoxidation von Kohlenwasserstoffen mit mindestens vier Kohlenstoffatomen mit Sauerstoff enthaltenden Gasen in Gegenwart einer flüchtigen Phosphorverbindung an einem Vanadium, Phosphor und Sauerstoff enthaltenden Katalysator in einer Rohrbündelraaktor-Einheit mit mindestens einer durch ein Wärmeträgermedium gekühlten Reaktionszone bei einer Temperatur im Bereich von 350 bis 500°C, **dadurch gekennzeichnet, dass** man in der, in Bezug auf die Edukt-Zufuhr, ersten Reaktionszone die Zulauftemperatur und/oder die Zulaufmenge des Wärmeträgermediums derart einstellt, dass die mittlere Temperatur des Wärmeträgermediums in der ersten Reaktionszone $T_{SB}$ (1. Zone), welche sich durch Mittelwertbildung aus der Zulauf temperatur und der Ablauftemperatur des Wärmeträgermediums ergibt, die Formeln (I) und (II)

$$T_{SB}(1.\ Zone) \leq T_D(1.\ Zone) - T_{Safety}(1 - Zone) \tag{I}$$

$$T_{SB,Amax}(1.\ Zone) - T_A(1.\ Zone) \leq T_{SB}(1.\ Zone) \leq$$

$$T_{SB,Amax}(1.\ Zone) + T_B(1.\ Zone) \tag{II}$$

erfüllt, wobei
$T_D$(1. Zone) für die Durchgehtemperatur der ersten Reaktionszone steht, wobei diese der mittleren Temperatur des Wärmeträgermediums $T_{SB}$(1. Zone) entspricht, bei der eine Erhöhung von einer um 1°C tieferen mittleren Temperatur des Wärmeträgermediums $T_{SB}$(1. Zone) - 1°C um 1°C auf $T_{SB}$ (1. Zone) eine Erhöhung der Heißpunkt-Tempezatur in der ersten Reaktionszone $T_{HS}$ (1. Zone) um 5°C bewirkt;
$T_{Safety}$ (1. Zone) für die Sicherheitstemperatur der ersten Reaktionszone steht und 1°C beträgt;
$T_{SB,Amax}$(1. Zone) für die mittlere Temperatur des Wärmeträgermediums in der ersten Reaktionszone steht, bei der im Bereich von $T_{SB}$(1. Zone) $\leq T_D$(1. Zone) das Maximum der Ausbeute an Maleinsäureanhydrid erreicht wird;
$T_A$(1. Zone) 20°C beträgt; und
$T_B$(1. Zone) 10°C beträgt.

2. Verfahren nach Anspruch 1, **dadurch gekennzeichnet, dass** $T_{Safety}$(1. Zone) in Formel (II) 2°C beträgt.

3. Verfahren nach den Ansprüchen 1 bis 2, **dadurch gekennzeichnet, dass** man eine Rohrbündelreaktonr-Einheit mit mindestens zwei durch ein Wärmeträgermedium gekühlten Reaktionszonen einsetzt.

4. Verfahren nach Anspruch 3, **dadurch gekennzeichnet, dass** man in der, in Bezug auf die Edukt-Zufuhr, zweiten Reaktionszone die Zulauftemperatur und/oder die Zulaufmenge des Wärmeträgermediums derart einstellt, dass die mittlere Temperatur des Wärmeträgermediums in der zweiten Reaktionszone $T_{SB}$(2. Zone), welche sich durch Mittelwertbildung aus der Zulauftemperatur und der Ablauftemperatur des Wärmeträgermediums ergibt, die Formeln (III) und (IV)

$$T_{SB}(2.\ Zone) \leq T_D(2.\ Zone) - T_{Safety}(2.\ Zone) \tag{III}$$

$$T_{SB,Amax}(2.\ Zone) - T_A(2.\ Zone) \leq T_{SB}(2.\ Zone) \leq$$

$$T_{SB,Amax}(2.\ Zone) + T_B(2.\ Zone) \tag{IV}$$

erfüllt, wobei
$T_D$(2. Zone) für die Durchgehtemperatrur der zweiten Reaktionszone steht, wobei diese der mittleren Temperatur des Wärmeträgermediums $T_{SB}$(2. Zone) entsprichst, bei der eine Erhöhung von einer um 1°C tieferen mittleren Temperatur des Wärmeträgermediums $T_{SB}$(2. Zone) - 1°C um 1°C auf $T_{SB}$(2. Zone) eine Erhöhung der Heißpunkt-Temperatur in der zweiten Reaktionszone $T_HS$ (2. Zone) um 5°C bewirkt;
$T_{Safety}$(2. Zone) für die Sicherheitstemperatur der zweiten Reaktionszone steht und 1°C beträgt;
$T_{SB,Amax}$ (2. Zone) für die mittlere Temperatur des Wärme trägermediums in der zweiten Reaktionszone steht, bei der im Bereich von $T_{SB}$(2. Zone) ≤ $T_D$(2. Zone) das Maximum der Ausbeute an Maleinsäureanhydrid erreicht wird; $T_A$(2. Zone) 10°C beträgt; und $T_B$(2. Zone) 10°C beträgt.

5.  Verfahren nach Anspruch 4, **dadurch gekennzeichnet, dass** $T_{Safety}$(2. Zone) in Formel (IV) 2°C beträgt.

6.  Verfahren nach den Ansprüchen 3 bis 5, **dadurch gekennzeichnet, dass** man die Zulauftemperatur und/oder die Zulaufmenge des Wärmeträgermediums der zweiten Reaktionszone derart einstellt, dass die Heißpunkt-Temperatur der zweiten Reaktionszone $T_{HS}$ (2. Zone) höher ist als die Heißpunkt-Temperatur der ersten Reaktionszone $T_{HS}$(1. Zone).

7.  Verfahren nach den Ansprüchen 1 bis 6, **dadurch gekennzeichnet, dass** man in mindestens einer der Reaktionszonen eine, hinsichtlich der Aktivität, strukturierte Katalysatorschüttung einsetzt.

8.  Verfahren nach den Ansprüchen 1 bis 7, **dadurch gekennzeichnet, dass** man als Kohlenwasserstoff n-Butan einsetzt.

9.  Verfahren nach den Ansprüchen 1 bis 8, **dadurch gekennzeichnet, dass** man als flüchtige Phosphorverbindung Tri-($C_1$- bis $C_4$-alkyl)-phosphat einsetzt.

10. Verfahren nach den Ansprüchen 1 bis 9, **dadurch gekennzeichnet, dass** man die heterogenkatalytische Gasphasenoxidation bei einem Druck von 0,1 bis 1 MPa abs durchführt.

**Claims**

1.  A process for preparing maleic anhydride by heterogeneously catalyzed gas-phase oxidation of hydrocarbons having at least four carbon atoms by means of oxygen-containing gases at from 350 to 500°C in the presence of a volatile phosphorus compound over a vanadium-, phosphorus- and oxygen-containing catalyst in a shell-and-tube reactor unit having at least one reaction zone cooled by means of a heat transfer medium, wherein the temperature and/or the amount of the heat transfer medium flowing into the first (relative to the feed direction) reaction zone are set so that the mean temperature of the heat transfer medium in the first reaction zone $T_{SB}$($1^{st}$ zone), which is calculated as the mean of the inflow temperature and the outflow temperature of the heat transfer medium, is in accordance with the formulae (I) and (II)

$$T_{SB}(1^{st}\ zone) \leq T_R(1^{st}\ zone) - T_{safety}(1^{st}\ zone) \tag{I}$$

$$T_{SB,Ymax}(1^{st}\ zone) - T_A(1^{st}\ zone) \leq T_{SB}\ (1^{st}\ zone) \leq$$

$$T_{SB,\ Ymax}(1^{st}\ zone) + T_B(1^{st}\ zone) \tag{II},$$

where
$T_R$($1^{st}$ zone) is the runaway temperature of the first reaction zone, which corresponds to the mean temperature of the heat transfer medium $T_{SB}$($1^{st}$ zone) at which an increase of 1°C from a 1°C-lower mean temperature of the heat transfer medium $T_{SB}$($1^{st}$ zone) - 1°C to $T_{SB}$($1^{st}$ zone) causes an increase of 5°C in the hotspot temperature in the first reaction zone $T_{HS}$($1^{st}$ zone);
$T_{Safety}$($1^{st}$ zone) is the safety temperature of the first reaction zone and has a value of 1°C;
$T_{SB,Ymax}$($1^{st}$ zone) is the mean temperature of the heat transfer medium in the first reaction zone at which the maximum maleic anhydride yield is achieved in the range $T_{SB}$($1^{st}$ zone) ≤ $T_R$($1^{st}$ zone);
$T_A$($1^{st}$ zone) is 20°C; and
$T_B$($1^{st}$ zone) is 10°C.

2.  A process as claimed in claim 1, wherein $T_{Safety}$($1^{st}$ zone) in the formula (II) is 2°C.

3.  A process as claimed in claim 1 or 2, wherein a shell-and-tube reactor unit having at least two reaction zones cooled by means of a heat transfer medium is used.

4. A process as claimed in claim 3, wherein the temperature and/or the amount of the heat transfer medium flowing into the second (relative to the feed direction) reaction zone are set so that the mean temperature of the heat transfer medium in the second reaction zone $T_{SB}(2^{nd}$ zone), which is calculated as the mean of the inflow temperature and the outflow temperature of the heat transfer medium, is in accordance with the formulae (III) and (IV)

$$T_{SB}(2^{nd}\ \text{zone}) \leq T_R(2^{nd}\ \text{zone}) - T_{Safety}(2^{nd}\ \text{zone}) \tag{III}$$

$$T_{SB,Ymax}(2^{nd}\ \text{zone}) - T_A(2^{nd}\ \text{zone}) \leq T_{SB}(2^{nd}\ \text{zone}) \leq$$

$$T_{SB,Ymax}(2^{nd}\ \text{zone}) + T_B(2^{nd}\ \text{zone}) \tag{IV},$$

where
$T_R(2nd\ zone)$ is the runaway temperature of the second reaction zone, which corresponds to the mean temperature of the heat transfer medium $T_{SB}(2nd\ zone)$ at which an increase of $1°C$ from a $1°C$-lower mean temperature of the heat transfer medium $T_{SB}(2^{nd}\ zone) - 1°C$ to $T_{SB}(2^{nd}\ zone)$ causes an increase of $5°C$ in the hotspot temperature in the second reaction zone $T_{HS}(2^{nd}\ zone)$;
$T_{Safety}(2^{nd}\ zone)$ is the safety temperature of the second reaction zone and has a value of $1°C$;
$T_{SB,Ymax}(2^{nd}\ zone)$ is the mean temperature of the heat transfer medium in the second reaction zone at which the maximum maleic anhydride yield is achieved in the range $T_{SB}(2^{nd}\ zone) \leq T_R(2^{nd}\ zone)$;
$T_A(2^{nd}\ zone)$ is $10°C$; and
$T_B(2^{nd}\ zone)$ is $10°C$.

5. A process as claimed in claim 4, wherein $T_{Safety}(2^{nd}\ zone)$ in the formula (IV) is $2°C$.

6. A process as claimed in any of claims 3 to 5, wherein the temperature and/or the amount of the heat transfer medium flowing into the second reaction zone are set so that the hotspot temperature of the second reaction zone $T_{HS}(2^{nd}\ zone)$ is higher than the hotspot temperature of the first reaction zone $T_{HS}(1^{st}\ zone)$.

7. A process as claimed in any of claims 1 to 6, wherein a catalyst bed which is structured in respect of its activity is used in at least one of the reaction zones.

8. A process as claimed in any of claims 1 to 7, wherein the hydrocarbon used is n-butane.

9. A process as claimed in any of claims 1 to 8, wherein the volatile phosphorus compound used is a tri($C_1$-$C_4$-alkyl) phosphate.

10. A process as claimed in any of claims 1 to 9, wherein the heterogeneously catalyzed gas-phase oxidation is carried out at a pressure of from 0.1 to 1 MPa abs.

**Revendications**

1. Procédé de préparation d'un anhydride d'acide maléique par oxydation en phase gazeuse à catalyse hétérogène d'hydrocarbures comportant au moins 4 atomes de carbone avec des gaz contenant de l'oxygène, en présence d'un composé de phosphore volatil sur un catalyseur contenant du vanadium, du phosphore et de l'oxygène dans une unité de réacteur à faisceau de tubes comportant au moins une zone réactionnelle refroidie par un milieu caloporteur à une température de l'ordre de 350 à 500°C, **caractérisé en ce que**, dans la première zone réactionnelle, par rapport à l'amenée de la matière de départ, on ajuste la température d'entrée et/ou la quantité d'entrée du milieu caloporteur de telle façon que la température moyenne du milieu caloporteur dans la première zone réactionnelle $T_{SB}$ ($1^{ère}$ zone), qui est obtenue par réalisation d'une moyenne de la température d'entrée et de la température de sortie du milieu caloporteur, réponde aux formules (I) et (II)

$$T_{SB} \text{ (1}^{\text{ère}} \text{ zone)} \leq T_D \text{ (1}^{\text{ère}} \text{ zone)} - T_{safety} \text{ (1}^{\text{ère}} \text{ zone)} \qquad \text{(I)}$$

$$T_{SB,Amax} \text{ (1}^{\text{ère}} \text{ zone)} - T_A \text{ (1}^{\text{ère}} \text{ zone)} \leq T_{SB} \text{ (1}^{\text{ère}} \text{ zone)} \leq$$

$$T_{SB,Amax} \text{ (1}^{\text{ère}} \text{ zone)} + T_B \text{ (1}^{\text{ère}} \text{ zone)} \qquad \text{(II)}$$

où

$T_D$ (1ère zone) représente la température de passage de la première zone réactionnelle, celle-ci correspondant à la température moyenne du milieu caloporteur $T_{SB}$ (1ère zone) pour laquelle une augmentation de 1°C d'une température moyenne, plus basse de 1°C, du milieu caloporteur $T_{SB}$ (1ère zone) - 1°C à la $T_{SB}$ (1ère zone) produit une augmentation de la température de point chaud dans la première zone réactionnelle $T_{HS}$ (1ère zone) de 5°C,

$T_{Safety}$ (1ère zone) représente la température de sécurité de la première zone réactionnelle et est de 1°C,

$T_{SB,Amax}$ (1ère zone) représente la température moyenne du milieu caloporteur dans la première zone réactionnelle pour laquelle, dans la gamme de $T_{SB}$ (1ère zone) $\leq T_D$ (1ère zone), le maximum du rendement en anhydride d'acide maléique est atteint,

$T_A$ (1ère zone) est de 20°C, et

$T_B$ (1ère zone) est de 10°C .

2. Procédé suivant la revendication 1, **caractérisé en ce que** $T_{Safety}$ (1ère zone) dans la formule (II) est de 2°C.

3. Procédé suivant les revendications 1 et 2, **caractérisé en ce qu'**on met en oeuvre une unité de réacteur à faisceau de tubes comportant au moins 2 zones réactionnelles refroidies par un milieu caloporteur.

4. Procédé suivant la revendication 3, **caractérisé en ce que**, dans la deuxième zone réactionnelle par rapport à l'amenée de matière de départ, on ajuste la température d'entrée et/ou la quantité d'entrée du milieu caloporteur de façon à ce que la température moyenne du milieu caloporteur dans la deuxième zone réactionnelle $T_{SB}$ (2ème zone), qui est obtenue par réalisation de la moyenne de la température d'entrée et de la température de sortie du milieu caloporteur, réponde aux formules (III) et (IV)

$$T_{SB} \text{ (2}^{\text{ème}} \text{ zone)} \leq T_D \text{ (2}^{\text{ème}} \text{ zone)} - T_{safety} \text{ (2}^{\text{ème}} \text{ zone)} \qquad \text{(III)}$$

$$T_{SB,Amax} \text{ (2}^{\text{ème}} \text{ zone)} - T_A \text{ (2}^{\text{ème}} \text{ zone)} \leq T_{SB} \text{ (2}^{\text{ème}} \text{ zone)} \leq$$

$$T_{SB,Amax} \text{ (2}^{\text{ème}} \text{ zone)} + T_B \text{ (2}^{\text{ème}} \text{ zone)} \qquad \text{(IV)}$$

où

$T_D$ (2ème zone) représente la température de passage de la deuxième zone réactionnelle, celle-ci correspondant à la température moyenne du milieu caloporteur $T_{SB}$ (2ème zone) pour laquelle une augmentation de 1°C d'une température moyenne, plus basse de 1°C, du milieu caloporteur $T_{SB}$ (2ème zone) - 1°C à la $T_{SB}$ (2ème zone) produit une augmentation de la température de point chaud dans la deuxième zone réactionnelle $T_{HS}$ (2ème zone) de 5°C,

$T_{Safety}$ (2ème zone) représente la température de sécurité de la deuxième zone réactionnelle et est de 1°C,

$T_{SB,Amax}$ (2ème zone) représente la température moyenne du milieu caloporteur dans la deuxième zone réactionnelle pour laquelle, dans la gamme de $T_{SB}$ (2ème zone) $\leq T_D$ (2ème zone), le maximum du rendement en anhydride d'acide maléique est atteint,

$T_A$ (2ème zone) est de 10°C et,

$T_B$ (2ème zone) est de 10°C.

5. Procédé suivant la revendication 4 **caractérisé en ce que** la $T_{Safety}$ (2ème zone) dans la formule (IV) est de 2°C.

6. Procédé suivant les revendications 3 à 5, **caractérisé en ce qu'**on ajuste la température d'entrée et/ou la quantité d'entrée du milieu caloporteur de la deuxième zone réactionnelle de façon à ce que la température de point chaud de la deuxième zone réactionnelle $T_{HS}$ (2ème zone) soit plus élevée que la température de point chaud de la première zone réactionnelle $T_{HS}$ (1ère zone).

**7.** Procédé suivant les revendications 1 à 6, **caractérisé en ce qu'**on met en oeuvre dans au moins une des zones réactionnelles un garnissage de catalyseur structuré du point de vue de l'activité.

**8.** Procédé suivant les revendications 1 à 7, **caractérisé en ce que**, comme hydrocarbure, on met en oeuvre du n-butane.

**9.** Procédé suivant les revendications 1 à 8, **caractérisé en ce que**, comme composé de phosphore volatil, on met en oeuvre du phosphate de tri- ($C_1$-$C_4$-alkyle).

**10.** Procédé suivant les revendications 1 à 9, **caractérisé en ce qu'**on effectue l'oxydation en phase gazeuse à catalyse hétérogène à une pression de 0,1 à 1 Mpa abs.

# FIG.1

# FIG.2

# FIG.3

# FIG.4

# FIG.5

# FIG.6

# FIG.7

# FIG.8